(19) Europäisches Patentamt
European Patent Office
Office européen des brevets

(11) Publication number: **0 242 052 B1**

(12) **EUROPEAN PATENT SPECIFICATION**

(45) Date of publication of patent specification: **21.04.93**

(21) Application number: **87302097.8**

(22) Date of filing: **11.03.87**

(51) Int. Cl.5: **C07D 493/22**, C07D 493/20, A61K 31/365, C12P 17/08, A01N 43/90, //(C07D493/22, 313:00,311:00,311:00,307:00), (C07D493/20,313:00,311:00, 311:00),(C12P17/00,C12R1:465)

(54) **Macrolide compounds.**

(30) Priority: **12.03.86 GB 8606110**
**12.03.86 GB 8606111**
**12.03.86 GB 8606112**
**12.03.86 GB 8606113**
**12.03.86 GB 8606114**
**12.03.86 GB 8606115**

(43) Date of publication of application:
**21.10.87 Bulletin 87/43**

(45) Publication of the grant of the patent:
**21.04.93 Bulletin 93/16**

(84) Designated Contracting States:
**AT BE CH DE ES FR GB IT LI NL SE**

(56) References cited:
**EP-A- 0 170 006**

(73) Proprietor: **AMERICAN CYANAMID COMPANY**
**One Cyanamid Plaza**
**Wayne New Jersey 07470(US)**

(72) Inventor: **Rudd, Brian A. M.**
**72 Exeter Road**
**Rayners Lane Harrow(GB)**
Inventor: **Fletton, Richard A.**
**12 St. Edmunds Avenue**
**Ruislip Middlesex(GB)**
Inventor: **Ward, John B.**
**33 Bournehall Road**
**Bushey Hertfordshire(GB)**
Inventor: **Noble, David**
**113 Stomp Road**
**Burnam Slough(GB)**
Inventor: **Porter, Neil**
**111 George V. Avenue**
**Pinner Middlesex(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 242 052 B1

**JOURNAL OF ANTIBIOTICS, vol. XXXVI, no. 8, August 1983; H.MISHIMA et al., pp. 980-984 H. MISHIMA et al.: "Milbemycins, a new family of macrolide antibiotics structure determination of milbemycins D,E,F,G, H,J and K."**

Inventor: **Lawrence, Gordon C.**
**20 Sands Farm Drive**
**Burnham Slough(GB)**
Inventor: **Noble, Hazel M.**
**113 Stomp Road**
**Burnham Slough(GB)**

(74) Representative: **Skailes, Humphrey John et al**
**Frank B. Dehn & Co. Imperial House 15-19**
**Kingsway**
**London WC2B 6UZ (GB)**

## Description

This invention relates to new antibiotic compounds and to processes for their preparation. More particularly it relates to antibiotic compounds which may be obtained by fermentation of Streptomyces microorganisms.

UK Patent Specification 2166436 and European Patent Specification 170006 describe the production of a class of substances, which we have designated Antibiotics S541, which may be isolated from the fermentation products of a novel Streptomyces sp. We have now found a further group of compounds with antibiotic activity which may be prepared by isolation from a culture of a microorganism of the genus Streptomyces as described herein. Compounds according to the invention have antibiotic activity as described below and also are of particular use as intermediates in the preparation of other compounds with antibiotic activity.

Thus, in one aspect of the invention we provide a group of related compounds having the formula (I)

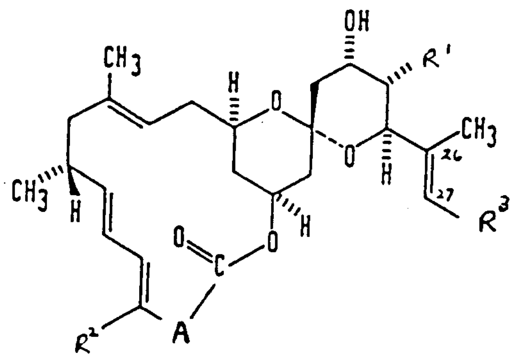

(I)

[wherein $R^1$ represents a methyl group;

$R^2$ represents a methyl group;

$R^3$ represents a methyl, ethyl or isopropyl group; and

-A- represents a ring which is either

(i) 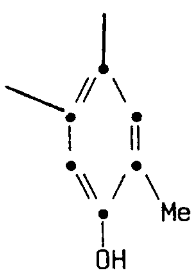 or (ii) 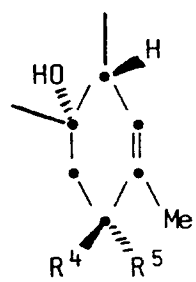

(where $R^4$ is a hydroxy or methoxy group and $R^5$ is a hydrogen atom or $R^4$ and $R^5$ together with the carbon atom to which they are attached represent a group C=O); or when -A- represents ring (ii) wherein $R^4$ and $R^5$ together with the carbon atom to which they are attached represent a group C=O then $R^2$ may also represent an aldehydo group;

or $R^2$ represents a hydroxymethyl group and -A- represents

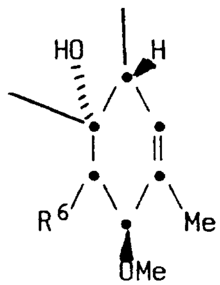

(where $R^6$ represents a hydrogen atom or a hydroxy group); with the provisos that when $R^2$ represents a methyl group and $R^4$ represents a methoxy group then $R^3$ cannot represent a methyl or isopropyl group and when $R^2$ represents a hydroxymethyl group and $R^6$ represents a hydroxy group then $R^3$ cannot represent a methyl group.

Compounds of the invention have antibiotic activity e.g. antihelminthic activity, for example against nematodes, and in particular, anti-endoparasitic and anti-ectoparasitic activity.

Ectoparasites and endoparasites infect humans and a variety of animals and are particularly prevalent in farm animals such as pigs, sheep, cattle, goats and poultry (e.g. chickens and turkeys), horses, rabbits, game-birds, caged birds, and domestic animals such as dogs, cats, guinea pigs, gerbils and hamsters. Parasitic infection of livestock, leading to anaemia, malnutrition and weight loss is a major cause of economic loss throughout the world.

Examples of genera of endoparasites infecting such animals and/or humans are Ancylostoma, Ascaridia, Ascaris, Aspicularis, Brugia, Bunostomum, Capillaria, Chabertia, Cooperia, Dictyocaulus, Dirofilaria, Dracunculus, Enterobius, Haemonchus, Heterakis, Loa, Necator, Nematodirus, Nematospiroides (Heligomoroides), Nippostrongylus, Oesophagostomum, Onchocerca, Ostertagia, Oxyuris, Parascaris, Strongylus, Strongyloides, Syphacia, Toxascaris, Toxocara, Trichonema, Trichostrongylus, Trichinella, Trichuris, Triodontophorus, Uncinaria and Wuchereria.

Examples of ectoparasites infecting animals and/or humans are arthropod ectoparasites such as biting insects, blowfly, fleas, lice, mites, sucking insects, ticks and other dipterous pests.

Examples of genera of such ectoparasites infecting animals and/or humans are Ambylomma, Boophilus, Chorioptes, Culliphore, Demodex, Damalinia, Dermatobia, Gastrophilus, Haematobia, Haematopinus, Haemophysalis, Hyaloma, Hypoderma, Ixodes, Linognathus, Lucilia, Melophagus, Oestrus, Otobius, Otodectes, Psorergates, Psoroptes, Rhipicephalus, Sarcoptes, Stomoxys and Tabanus.

The antibiotic activity of compounds of the invention may be demonstrated by their activity against free living nematodes e.g. Caenorhabditis elegans.

Compounds of the invention are also of use as anti- fungals, for example, against strains of Candida sp. such as Candida albicans and Candida glabrata and against yeast such as Saccharomyces carlsbergensis.

Compounds of the invention are also of use in combating insect, acarine and nematode pests in agriculture, horticulture, forestry, public health and stored products. Pests of soil and plant crops, including cereals (e.g. wheat, barley, maize and rice), cotton, tobacco, vegetables (e.g. soya), fruit (e.g. apples, vines and citrus) as well as root crops (e.g. sugarbeet, potatoes) may usefully be treated. Particular examples of such pests are fruit mites and aphids such as Aphis fabae, Aulacorthum circumfleum, Myzus persicae, Nephotettix cincticeps, Nilparvata lugens, Panonychus ulmi, Phorodon humuli, Phyllocoptruta oleivora, Tetranychus urticae and members of the genera Trialeuroides; nematodes such as members of the genera Aphelencoides, Globodera, Heterodera, Meloidogyne and Panagrellus; lepidoptera such as Heliothis, Plutella and Spodopters; grain weevils such as Anthonomus grandis and Sitophilus granarius; flour beetles such as Tribolium castaneum; flies such as Musca domestica; fire ants; leaf miners; Pear psylla; Thrips tabaci; cockroaches such as Blatella germanica and Periplaneta americana and mosquitoes such as Aedes aegypti.

According to the invention we therefore provide compounds of formula (I) as defined above, which may be used as antibiotics. In particular, they may be used in the treatment of animals and humans with endoparasitic, ectoparasitic and/or fungal infections and in agriculture, horticulture, or forestry as pesticides to combat insect, acarine and nematode pests. They may also be used generally as pesticides to combat or control pests in other circumstances, e.g. in stores, buildings or other public places or location of the pests. In general the compounds may be applied either to the host (animal or human or plants or vegetation) or a locus thereof or to the pests themselves.

Compounds of the invention may be formulated for administration in any convenient way for use in veterinary or human medicine and the invention therefore includes within its scope pharmaceutical

compositions comprising a compound in accordance with the invention adapted for use in veterinary or human medicine. Such compositions may be presented for use in conventional manner with the aid of one or more suitable carriers or excipients. The compositions of the invention include those in a form especially formulated for parenteral (including intramammary administration), oral, rectal, topical, implant, ophthalmic, nasal or genito-urinary use. Suitable formulations for use in veterinary or human medicine are as described in our UK Patent Specification 2166436.

The total daily dosages of compounds of the invention employed in both veterinary and human medicine will suitably be in the range 1-2000µg/kg bodyweight, preferably from 50-1000 µg/kg and these may be given in divided doses, e.g. 1-4 times per day.

The compounds according to the invention may be formulated in any convenient way for horticultural or agricultural use and the invention therefore includes within its scope compositions comprising a compound according to the invention adapted for horticultural or agricultural use. Suitable formulations for use in horticulture or agriculture are as described in our UK Patent Specification 2166436.

In the formulations, the concentration of active material is generally from 0.01 to 99% and more preferably between 0.01% and 40% by weight.

Commercial products are generally provided as concentrated compositions to be diluted to an appropriate concentration, for example from 0.001 to 0.0001% by weight, for use.

For use in horticulture and agriculture or for use in veterinary medicine it may be desirable to use whole fermentation broth, as a source of the compounds of the invention. It may be suitable to use dried broth (containing mycelia) or to use lysed mycelia, live or dead mycelia separated from the broth using solid/liquid separation or evaporation techniques or to use the fermentation broth remaining after separation of the mycelia. If desired the mycelia may be pasteurised or more preferably, dried e.g. by spray drying or roller drying. If desired the broth or mycelia may be formulated into compositions including conventional inert carriers, excipients or diluents as described above.

It will be appreciated from the above that in general compounds of the invention may be used to combat infections or infestations by applying to the organism responsible for the infection or infestation or a location thereof an effective amount of one or more of said compounds.

The antibiotic compounds of the invention may be administered or used in combination with other active ingredients.

In particular, the antibiotic compounds of the invention may be used together with Antibiotics S541 compounds or with other antibiotic compounds. This may occur, for example, where whole fermentation broth is used without prior separation of the compounds of the invention or where crude fermentation products are reacted according to a process of the invention without prior or subsequent separation; this may be preferable for example in agricultural use of the compounds, where it is important to maintain low production costs.

According to a further aspect of the invention we provide a process for the production of the compounds of the invention as defined previously which comprises the step of cultivating an organism of the genus Streptomyces capable of producing at least one of the compounds of the invention whereby at least one of said compounds is produced, and if desired isolating said compound therefrom.

Microorganisms capable of producing the compounds of the invention may readily be identified by using a small scale test employing the nematode Caenorhabditis elegans, for example by adding a test sample [obtained from fermentation of the microorganism] to a suspension of the nematode and examining the consequent effect on nematode viability.

The organism of the genus Streptomyces will preferably be of the species Streptomyces thermoarchaensis or Streptomyces cyaneogriseus noncyanogenus. Particular examples of suitable strains include Streptomyces thermoarchaensis NCIB 12015 [deposited 10th September, 1984], Streptomyces thermoarchaensis, NCIB 12111, NCIB 12112, NCIB 12113, NCIB 12114 [all deposited 26th June, 1985], Streptomyces thermoarchaensis NCIB 12212 and NCIB 12334 [deposited 6th March 1986 and 15th September 1986 respectively in the permanent culture collection of the National Collections of Industrial and Marine Bacteria, Torry Research Station, Aberdeen, United Kingdom] and Streptomyces cyaneogriseus noncyanogenus NRRL 15773 [deposited 3rd May 1984] and mutants of all these strains. Streptomyces thermoarchaensis NCIB 12212 and mutants thereof form a further aspect of the invention. Streptomyces thermoarchaensis NCIB 12212 has substantially similar essential characteristics to those described for Streptomyces thermoarchaensis NCIB 12015 in UK Patent Specification 2166436.

According to a still further aspect of the invention we provide the genetic material of Streptomyces thermoarchaensis NCIB 12212 and mutants thereof that participates in the synthesis of compounds of formula (I). Such material may be obtained using conventional genetic engineering techniques including those outlined by D A Hopwood in 'Cloning genes for Antibiotic Biosynthesis in Streptomyces Spp.:

Production of a hybrid antibiotic' p409-413 in Microbiology 1985, Ed. L. Lieve, American Society of Microbiology, Washington D.C. 1985. Such techniques may be used in a similar manner to that described previously for cloning antibiotic biosynthetic genes, including the biosynthetic genes for actinorhodin (Malpartida, F. and Hopwood, D. A. 1984, Nature 309, p462-464), erythromycin (Stanzak, R. et al, 1986, Biotechnology, 4, p229-232) and an important enzyme involved in penicillin and cephalosporin production in Acremonium chrysogenum (Sansom, S.M. et al, 1985, Nature, 318, p191-194). The Streptomyces thermoarchaensis genetic material so obtained may be used, for example, for strain improvement, for production of biosynthetic enzymes for in vitro applications, or for generating novel antibiotics by introduction of such material into organisms other than Streptomyces thermoarchaensis.

Mutants of the above strains may arise spontaneously or may be produced by a variety of methods including those outlined in Techniques for the Development of Micro-organisms by H.I.Adler in 'Radiation and Radioisotopes for Industrial Microorganisms', Proceedings of the Symposium, Vienna 1973, p241, International Atomic Energy Authority. Such methods include ionising radiation, chemical methods e.g. treatment with N-methyl-N'- nitro-N-nitrosoguanidine (NTG); heat; genetic techniques, such as recombination, transduction, transformation, lysogenisation and lysogenic conversion, and selective techniques for spontaneous mutants.

The production of the compounds of the invention by fermentation of a suitable Streptomyces organism may be effected by conventional means i.e. by culturing the Streptomyces organism in the presence of assimilable sources of carbon, nitrogen and mineral salts according to the methods described in UK Patent Specification 2166436 and European Patent Specification 170006.

Where it is desired to separate the compounds of the invention from the whole fermentation this may be carried out by conventional isolation and separation techniques. The compounds according to the invention are predominantly contained in the mycelia of the cells, but may also be found in the fermentation broth and the isolation techniques may also be applied to the fermentation broth either before or after clarification. It will be appreciated that the choice of isolation techniques may be varied widely.

The compounds of the invention may be isolated, separated and purified according to the methods described in UK Patent Spcification 2166436 and European Patent Specification 170006.

The compounds of the invention may be used at levels of purity appropriate to their intended use. For use in human medicine, purities of at least 90%, preferably greater than 95%, are desirable. For veterinary or agricultural or horticultural use, lower purities will suffice, for example 50% or lower.

Compounds according to the invention are also particularly useful as intermediates for the production of other active compounds.

Thus we also provide a process for the production of a compound of formula (II)

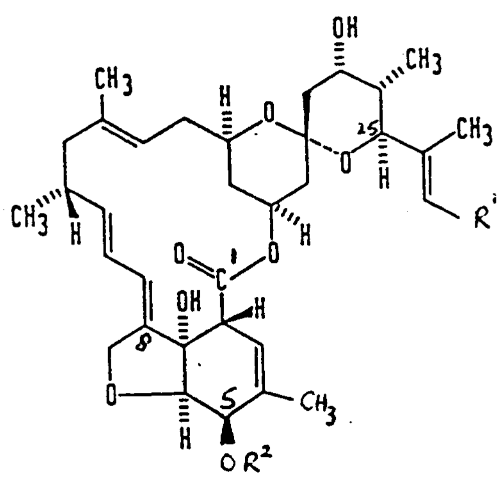

(II)

(wherein $R^1$ is a methyl, ethyl or isopropyl group and $R^2$ is a hydrogen atom or a methyl group) which comprises cultivating a suitable Streptomyces organism in the presence of a suitable compound of formula (I), whereby a compound of formula (II) is produced, and if desired isolating the said compound.

The process is particularly suitable for the production of the compound of formula (II) wherein $R^1$ is an isopropyl group and $R^2$ is a hydrogen atom. Compounds of formula (I) wherein $R^1$ and $R^2$ are methyl groups, $R^3$ is an isopropyl group and-A-represents

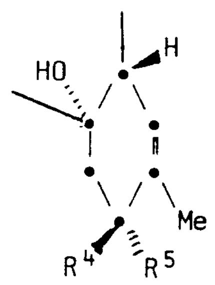

(where $R^4$ is a hydroxy group and $R^5$ is a hydrogen atom or $R^4$ and $R^5$ together with the carbon atom to which they are attached represent C=O) are particularly useful for the preparation of the compound of formula (II) wherein $R^1$ is an isopropyl group and $R^2$ is a hydrogen atom.

Compounds of formulae (II) are of particular interest for use as antibiotics as described above for the compounds of the invention and are described in our UK Patent Specification 2166436.

Suitable Streptomyces microorganisms for use in this aspect of the invention include strains of Streptomyces thermoarchaensis, Streptomyces cyaneogriseus noncyanogenus, Streptomyces avermitilis and Streptomyces hygroscopicus subsp. aureolacrimosus. Preferred strains include Streptomyces thermoarchaensis NCIB 12213, [deposited (6th March 1986) in the permanent culture collection of the National Collections of Industrial and Marine Bacteria, Torry Research Station, Aberdeen, United Kingdom] and mutants thereof Streptomyces avermitilis ATCC 31272 and Streptomyces hygroscopicus subsp. aureolacrimosus FERM P 1438.

Streptomyces thermoarchaensis NCIB 12213 and mutants thereof form another aspect of the invention. Streptomyces thermoarchaensis NCIB 12213 has substantially similar essential characteristics to those described for Streptomyces thermoarchaensis NICB 12015 in UK Patent Specification 2166436.

According to a still further aspect of the invention we provide the genetic material of Streptomyces thermoarchaensis NCIB 12213 and mutants thereof that participate in the synthesis of compounds of formula (II) from compounds of formula (I). Such material may be obtained using the conventional genetic engineering techniques previously described.

Mutants of Streptomyces thermoarchaensis NCIB 12213 may arise spontaneously or may be produced by a variety of methods including those described above for obtaining mutants of other Streptomyces thermoarchaensis strains.

Suitable Streptomyces microorganisms for use in this aspect of the invention may be identified by preliminary small scale tests designed to demonstrate ability of a microorganism to convert e.g. compounds of formula (I) to compound of formula (II).

The Streptomyces microorganism may be cultivated using the cultivation conditions described above for the cultivation of Streptomyces strains producing the compounds of the invention. The starting compound, in a suitable solvent [e.g. a water miscible organic solvent such as an alcohol, e.g. methanol or propan-2-ol, a diol e.g. propan-1,2-ol or butan-1,3-ol, a ketone, e.g. acetone, a nitrile e.g. acetonitrile, an ether, e.g. tetrahydrofuran or dioxan, a substituted amide e.g. dimethylformamide or a dialkylsulphoxide e.g. dimethylsulphoxide] may be added at the beginning of the cultivation, or more usually, when the growth of the microorganism is under way, e.g. 2 to 4 days after the start of the cultivation. Mixtures of different starting materials may be used and the invention is intended to cover the use of such mixtures.

Once the starting material has been added to the culture, usually with gentle mixing, the cultivation is continued such that the desired product is accumulated. The presence of the product in the fermentation broth may be determined by monitoring extracts of the broth by high performance liquid chromatography, and uv spectroscopy at 238nm.

The products may be isolated from the whole fermentation broth by conventional isolation and separation techniques such as those described above for the isolation and separation of compounds of the invention.

The following Examples illustrate the invention. The following abbreviations are used : tlc - thin layer chromatography (using Merck 5735 silica 60 plates and developed with $CHCl_3$:ethyl acetate (3:1) unless otherwise specified); CCM - column chromatograpy using Merck 7734 silica 60 (200 x 4cm column unless otherwise specified) packed and eluted with $CHCl_3$:ethyl acetate (3:1) unless otherwise specified; hplc - high performance liquid chromatography; PE - petroleum ether (b.p. 60-80°C unless otherwise specified); L - litre; EA - ethyl acetate.

Temperatures are in °C. Arkasoy, Spherisorb, Sephadex, Altex, Finnigan, Sturzal, Dicalite, Rettenmeier, Polysobate, and Sypetonic are trade marks or trade names.

Media A and B referred to in the Examples are :

Medium A

| | $gL^{-1}$ |
|---|---|
| D-Glucose | 15.0 |
| Glycerol | 15.0 |
| Soya Peptone | 15.0 |
| NaCl | 3.0 |
| Calcium carbonate | 1.0 |

Distilled water to 1 L, pH adjusted to pH 7.0 with aqueous NaOH before autoclaving.

Medium B

| | $gL^{-1}$ |
|---|---|
| D-Glucose | 2.5 |
| Malt daxtrin MD 30E (Roquette (UK)Ltd) | 25.0 |
| Arkasoy 50 (British Arkady Co.Ltd) | 12.5 |
| Molasses | 1.5 |
| $K_2HPO_4$ | 0.125 |
| Calcium carbonate | 1.25 |
| MOPS (3-(N-morpholino)propane-sulphonic acid) | 21.0 |

Distilled water to 1 L, pH adjusted to 6.5 with 5N NaOH before autoclaving.

In the following examples reference is made to Factor B. Factor B is a known compound described in UK Patent Specification 2166436.

Example 1

Spores of Streptomyces thermoarchaensis NCIB 12015 were inoculated onto agar slants made up of the following ingredients:

| | $gL^{-1}$ |
|---|---|
| Yeast extract (Oxoid L21) | 0.5 |
| Malt extract (Oxoid L39) | 30.0 |
| Mycological Peptone (Oxoid L40) | 5.0 |
| Agar No. 3 (Oxoid L13) | 15.0 |

Distilled water to 1L, pH approximately 5.4.
and incubated at 28°C for 10 days. The mature slant was then covered with a 10% glycerol solution (6ml) and scraped with a sterile tool to loosen the spores and mycelium. 0.4ml of the resulting spore suspension was used to inoculate a 250ml Erlenmeyer flask containing Medium A (50ml). The flask was incubated at 28°C for 2 days on a shaker rotating at 250 rpm with a 50mm diameter orbital motion. Portions (8ml) were then used to inoculate each of two 2L flat-bottomed flasks, each containing 400ml of the same medium, before incubation under the same conditions for 2 days. The contents of both flasks were then used to inoculate a fermenter vessel (70L) containing Medium B (40L) supplemented with polypropylene 2000 (0.06% v/v) instead of Silicone 525. Polypropylene 2000 was added as required throughout the fermentation to control foaming. The fermentation was carried out at 28°C, with agitation and aeration sufficient to

maintain a dissolved oxygen level of greater than 30% saturation. After 24 hours of fermentation, a portion of broth (9L) was transferred to a fermenter (700L) containing medium (450L) made up as follows:

| | $gL^{-1}$ |
|---|---|
| D-glucose | 2.8 |
| Malt Dextrin (MD30E) | 27.8 |
| Arkasoy 50 | 13.9 |
| Molasses | 1.7 |
| $K_2HPO_4$ | 0.14 |
| $CaCO_3$ | 1.39 |
| Silicone 525 (Dow Corning) | 0.06% (v/v) |
| Adjusted to pH 6.5 before sterilisation. | |

The fermentation was carried out at 28°C with agitation and aeration. Polypropylena 2000 antifoam was added as required. The pH was controlled down to 7.2 with the addition of $H_2SO_4$. The fermentation was harvested after 5 days.

The broth (450L) was clarified by centrifugation and the residual supernatant was displaced with water (20L). The recovered cells (25.5kg) were stirred for 1 hour in sufficient methanol to give a total volume of 75L. The suspension was filtered and the solid residue was re-extracted with methanol (35L) and filtered. The combined filtrate (87L) was diluted with water (40L) and extracted with PE. After 30 min. the phases were separated by centrifugation and the lower methanol phase was re-extracted with PE (30L) after the addition of water (40L). After separation the lower phase was again extracted with PE (30L). The combined PE phases (85L) were concentrated by three passes through a Pfaudler 8.8-12v-27 wiped-film evaporator (vapour pressure 0.1 bar, vapour temperature 20°, steam temperature 127°). The concentrate (9L) was dried with sodium sulphate (2kg) and further concentrated under reduced pressure at 40° in a rotary film evaporator. The oily residue (130g) was dissolved in $CHCl_3$ to give 190ml and this was subjected to CCM [column packed and washed (500ml) in $CHCl_3$] fractions of approximately 40ml being collected after a forerun of 1,400ml.

Fractions 32-46 were combined and evaporated to yield an oil (21.2g). Fractions 47-93 were combined and evaporated to give an oil (20.1g) which was dissolved in $CHCl_3$:EA (3:1) to 50ml, and subjected to CCM, fractions of approximately 40ml being collected after a forerun of 1,400 ml. Fractions 22-36 were combined and evaporated to give an oil (3.1g) which was added to the oil obtained from fractions 32-46 from the first column. The combined oils were dissolved in boiling methanol (4ml) which was then added to hot propan-2-ol (20ml) to yield on standing crystals of the compound of formula (VI) in which $R^1$ and $R^2$ is each a methyl group (hereinafter referred to as Factor B).

Mother liquor after crystallisation of Factor B was evaporated to yield an oil which was dissolved in an equal volume of $CH_2Cl_2$ and loaded onto a column (30x2.2cm) of Merck Kieselgel 60 (70-230 mesh ASTM, Art. No. 7734) packed in $CH_2Cl_2$. The bed was washed with $CH_2Cl_2$ (2 bed volumes) and eluted with $CHCl_3$:EA (3:1) (2 bed volumes). Evaporation of the eluate yielded an oil which was dissolved in methanol and subjected to preparative hplc on Spherisorb S5-ODS2 (250mmx20mm, Phase Sep.Ltd.). The sample (5ml) was pumped onto the column over a period of 1 minute and the column was eluted with acetonitrile:water (7:3) under the following conditions:

| Time (mins) | Flow (ml/min) |
|---|---|
| 0.00 | 0.00 ) Injection |
| 1.00 | 0.00 ) time |
| 1.10 | 30.00 |
| 39.90 | 30.00 |
| 40.00 | 35.00 |
| 75.00 | 35.00 |

Material eluting from the hplc column was monitored by uv spectroscopy at 238nm.

(a) Evaporation of the combined fractions which contained material with a peak which began to elute after 44.7 minutes yielded an extract which was loaded onto a column (35x2.2cm i.d.) of Sephadex LH20 in acetonitrile. The column was developed with acetonitrile the fractions being monitored by analytical

hplc [Spherisorb S5ODS-2 (10cm x 0.46cm) in acetonitrile (65 parts $^{v}/_{v}$) made up to 100 parts $^{v}/_{v}$ with 0.1M $NH_4H_2PO_4$ and a flow rate of 3ml/min and detection at 238nm.] Those fractions containing a material with a retention ratio of 2.385 relative to Factor B when examined by analytical hplc were combined. Evaporation of these fractions yielded a foam which was dissolved in acetone (0.2ml), diluted with cyclohexane (1ml) and lyophilised to a colourless solid (23mg). The solid was the compound of formula (I) in which $R^1$ is methyl, $R^2$ is methyl, $R^3$ is isopropyl and -A- is

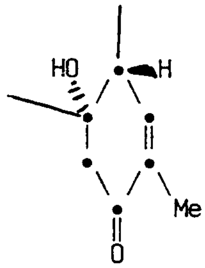

and had the following characteristics:

(i) A low resolution E.I. spectrum gave a molecular ion at m/z 596, consistent with a molecular formula of $C_{36}H_{52}O_7$, and also fragment ions at m/z 578, 560, 542, 408, 265, 247, 245, 237, 227 and 219.

(ii) It has a characteristic I.R. spectrum in bromoform (c = 1%) including peaks at about 3480 (OH), 1706 (ester) 1674 (conjugated ketone) and 994cm$^{-1}$(C-O).

(iii) It has a UV spectrum in methanol (c = 0.001%) showing a $\lambda_{max}$ of 236.5nm $E_1^1$ 483.

(iv) A 200 MHz proton nmr spectrum of a solution in deuterochloroform includes signals [$\delta$ values with multiplicities, coupling constants (Hz) and integration values in parentheses] centered at about:

| | |
|---|---|
| 6.39 (narrow m;1H) | 1.75 (s;3H) |
| 6.22 (d15;1H) | 1.59 (s;3H) |
| 6.01 (dd15,11;1H) | 1.57 (s;3H) |
| 2.75 (d16;1H) | 0.99 (d7;6H) |
| 2.46 (d16;1H) | 0.93 (d7;3H) |
| 1.85 (narrow m;3H) | 0.79 (d7;3H) |

(b) Evaporation of the combined fractions which contained material with a peak which began to elute after 68 minutes yielded a solid (30mg). This was extracted with acetonitrile and the extract was loaded onto a column (35x2.2cm i.d.) of Sephadex LH20 in acetonitrile. The column was developed with acetonitrile the fractions being monitored by analytical hplc [Spherisorb S5ODS-2 (10cm x 0.46cm) in acetonitrile (65 parts v/v) made up to 100 parts v/v with 0.1M $NH_4H_2PO_4$ and a flow rate of 3ml/min and detection at 238nm.] Those fractions containing a material with a retention ratio of 3.773 relative to Factor B when examined by analytical hplc were combined and evaporated to yield a colourless solid (6mg). The solid was the compound of formula (I) in which $R^1$ is methyl, $R^2$ is methyl, $R^3$ is isopropyl and A is

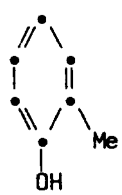

$^1$H NMR SPECTRUM ($CDCl_3$ solution)

A 250MH$_1$ $^1$H NMR spectrum includes signals centered at about

| | |
|---|---|
| δ 0.80 (d6;3H) | 2.06 (s;3H) |
| 0.95 (d6;3H) | 2.22 (2;3H) |
| 1.01 (d6;3H) | 5.69 (d12;1H) |
| 1.02 (d6;3H) | 6.12 (dd15,12;1H) |
| 1.58 (s;3H) | 6.61 (s;1H) |
| 1.62 (s;3H) | 7.38 (s;1H) |

I.R. SPECTRUM (bromoform solution)

Shows absorption bands at 3420 (OH), 1690 (conjugated $CO_2R$) and 994cm$^{-1}$ (C-O)

U.V. SPECTRUM

The spectrum of a ca 0.002% solution in methanol shows bands at 247nm (max) $E_1^1$ 475m 274nm (inf) $E_1^1$ 222.

MASS SPECTRUM

An E.I. mass spectrum gave a molecular ion at 578 nominal mass.

Example 2

A 0.4ml spore suspension of Streptomyces thermoarchaensis NCIB 12212 [prepared in a similar manner to the spore suspension used in Example 1 except using 20% glycerol solution] was used to inoculate a 250ml Erlenmeyer flask containing Medium B (50ml). This culture was incubated at 28°C for 2 days on a rotary shaker operating at 250 rev/min with a 50mm throw. 2% (v/v) portions of this 2 day developed culture were used to inoculate further 250ml Erlenmeyer flasks containing Medium B (50ml). The flasks were incubated at 28°C for 2 days on a rotary shaker operating at 250 rev/min. with a 50mm throw.

80ml of the shake flask inoculum were used to inoculate a 7L fermenter containing 4 L of medium made up as follows:

| | gL$^{-1}$ |
|---|---|
| Glucose | 41.25 |
| Arkasoy 50 | 18.8 |
| Beet Molases | 2.25 |
| $K_2HPO_4$ | 0.188 |
| $CaCO_3$ | 1.88 |

Made up in distilled water and pH adjusted to pH 6.5 before autoclaving.

The fermentation was carried out at 35°C. The culture was stirred at 500 rev/min and aerated at 3L/min. The pH was controlled down to pH 7.4 with acetic acid. The fermentation was harvested at 114h, to give 2L of culture fluid from which the cells were removed by centrifugation and then stored frozen.

The frozen cells were stirred with methanol 0.4L at 4° for 16 h, centrifuged (4200 r.p.m., 4°, 30 min) and the supernatant (500ml) was decanted. After the addition of water (250ml) the solution was extracted with hexane (1x500ml, 2x250ml) and the combined hexane extracts were dried over magnesium sulphate and evaporated to a mobile oil (10g). The oil was dissolved in hexane (100ml) and extracted with propane-1,2-diol (3x50ml). The combined diol extracts were washed with hexane (50ml), then diluted with EA (300ml) and washed with water (3x150ml) to remove diol. The EA solution was dried over magnesium sulphate and evaporated to a yellow solid (2.0g).

The solid was examined by hplc in combination with mass spectroscopy on a column (250x2mm) of Alfex OD52 in a 3:1 acetonitrile:water solvent system at a flow rate of 0.33ml/min. The column effluent was

interfaced with a Finnigan moving belt to a Finnigan MAT 4500 mass spectrometer under E.I. conditions and the mass spectra of materials was recorded. Compounds identified include (i) the compound of formula (I) in which $R^1$ is methyl, $R^3$ is ethyl and -A- is

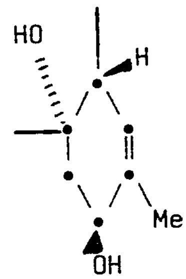

(low resolution E.I. spectrum gave fragment ions at m/z 566, 548, 530, 411, 264, 251, 233, 223 and 205) and (ii) the compound of formula (I) in which $R^1$ is methyl, $R^2$ is methyl, $R^3$ is methyl and -A- is

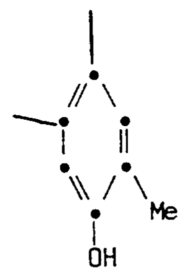

(low resolutioon E.I. spectrum gave fragment ions at m/z 532, 514, 191, 209, 219, 237, 227 and 245)

The solid was dissolved in acetonitrile (12ml) and water (4ml) was added to this solution. Portions (2ml) were chromatographed on a column (250x20mm) of Spherisorb S5-ODS2 in a solvent of acetonitrile (700 parts) made up to 1000 parts with water and a flow rate of 40ml/min. Materials eluting from the column were detected with a u.v. monitor at 238nm.

(a) Fractions eluting between 840 to 950 sec. from repeat injections were combined and evaporated to yield 215mg of the compound of formula (I) in which $R^1$ is methyl, $R^2$ is methyl, $R^3$ is methyl and -A- is

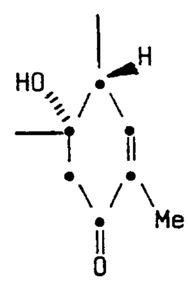

as a colourless solid. The compound had the following characteristics:

(i) E.I. mass spectroscopy yielded a molecular ion at m/z 568.3397 corresponding to a molecular formula of $C_{34}H_{48}O_7$.A low resolution E.I. spectrum gave fragment ions at m/z 550, 532, 472, 466, 245, 237, 227, 219, 209 and 191.

(ii) It has a characteristic I.R. spectrum in bromoform (c = 1%) including peaks at about 3500 (OH), 1710 (ester) 1678 (conjugated ketone) and 994cm$^{-1}$(C-O).

(iii) It has a UV spectrum in methanol (c = 0.002%) showing a $\lambda_{max}$ of 236.5nm $E_1^1$ 516.

(iv) A 200 MHz proton nmr spectrum of a solution in deuterochloroform includes signals [δ values with multiplicities, coupling constants (Hz) and integration values in parentheses] centered at about:

| | |
|---|---|
| 6.4 (broad S;1H) | 1.76 (s;3H) |
| 6.21 (d11;1H) | 1.63 (d6;3H) |
| 6.02 (dd15,11;1H) | 1.59 (s;3H) |
| 2.75 (d16;1H) | 1.57 (s;3H) |
| 2.46 (d16;1H) | 1.00 (d6;3H) |
| 1.88 (s;3H) | 0.78 (d7;3H) |

(b) Fractions eluting between 1200 to 1290 sec from repeat injections were combined and evaporated to yield 84mg of the compound of formula (I) in which $R^1$ is methyl, $R^2$ is methyl, $R^3$ is ethyl and -A- is

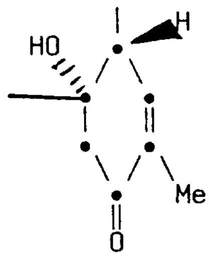

as a colourless solid. The compound had the following characteristics:

(i) E.I. mass spectroscopy yielded a molecular ion at m/z 582.3530 corresponding to a molecular formula of $C_{35}H_{50}O_7$.A low resolution E.I. spectrum gave fragment ions at m/z 564, 546, 528, 472, 466, 251, 245, 233, 227, 223 and 205.

(ii) It has a characteristic I.R. spectrum in bromoform ($\underline{c}$ = 1%) including peaks at about 3500 (OH), 1710 (ester) 1678 (conjugated ketone) and 994cm-1(C-O).

(iii) It has a UV spectrum in methanol ($\underline{c}$ = 0.001%) showing a $\lambda_{max}$ of 236.5nm $E_1^1$ 515.

(iv) A 200 MHz proton nmr spectrum of a solution in deuterochloroform includes signals [δ values with multiplicities, coupling constants (Hz) and integration values in parentheses] centered at about:

| | |
|---|---|
| 6.38 (narrow m;1H) | 1.86 (narrow m;3H) |
| 6.21 (d11;1H) | 1.76 (s;3H) |
| 5.99 (dd15;11;1H) | 1.00 (d6;3H) |
| 2.76 (d16;1H) | 0.97 (t7;3H) |
| 2.46 (d16;1H) | 0.80 (d7;3H) |

(c) Fractions eluting between 580 to 650 sec. from repeat injections were combined and evaporated to yield 234mg of the compound of formula (I) in which $R^1$ is methyl, $R^2$ is methyl, $R^3$ is methyl and -A- is

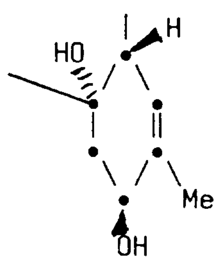

as a colourless solid. The compound had the following characteristics:

(i) E.I. mass spectroscopy yielded a molecular ion at m/z 570.3536 corresponding to a molecular formula of $C_{34}H_{50}O_7$.A low resolution E.I. spectrum gave fragment ions at m/z 552, 534, 468, 411, 264, 237, 219, 209 and 191.

(ii) It has a characteristic I.R. spectrum in bromoform ($\underline{c}$ = 1%) including peaks at about 3490 (OH), 1706 (ester) and 992cm$^{-1}$(C-O).

(iii) It has a UV spectrum in methanol (c = 0.002%) showing 236nm (inf) $E_1^1$ 435; 241nm (max) $E_1^1$ 459; 247nm (inf) $E_1^1$ 357.

(iv) A 200 MHz proton nmr spectrum of a solution in deuterochloroform includes signals [δ values with multiplicities, coupling constants (Hz) and integration values in parentheses] centered at about:

| | |
|---|---|
| 6.18 (d14;1H) | 1.84 (s;3H) |
| 6.02 (dd14,11;2H) | 1.63 (d6;3H) |
| 5.27 (broadened s;1H) | 1.57 (s;6H) |
| 1.71 (s;3H) | 1.00 (d6;3H) |
| 4.46 (t6;1H) | 0.78 (d7;3H) |
| 3.41 (m;1H) | |

(d) Fractions eluting between 1060 to 1130 sec from repeat injections were combined and evaporated to yield 98mg of the compound of formula (I) in which $R^1$ is methyl, $R^2$ is methyl, $R^3$ is isopropyl and A is

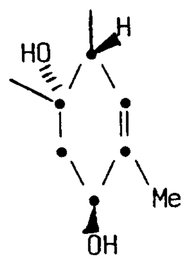

as a colourless solid. The compound had the following characteristics:

(i) E.I. mass spectroscopy yielded a molecular ion at m/z 598.3854 corresponding to a molecular formula of $C_{36}H_{54}O_7$.

A low resolution E.I. spectrum gave fragment ions at m/z 580, 562, 468, 411, 265, 264, 247, 237 and 219.

(ii) It has a characteristic I.R. spectrum in bromoform (c = 1%) including peaks at about 3490 (OH), 1706 (ester) and 994cm$^{-1}$(C-O).

(iii) It has a UV spectrum in methanol (c = 0.002%) showing 236nm (inf) $E_1^1$ 410, 241nm (max) $E_1^1$ 433.

(iv) A 200 MHz proton nmr spectrum of a solution in deuterochloroform includes signals [δ values with multiplicities, coupling constants (Hz) and integration values in parentheses] centered at about:

| | |
|---|---|
| 6.20 (d11;1H) | 1.59 (s;3H) |
| 6.04 (dd14;11;1H) | 1.57 (s;3H) |
| 5.27 (broad s;1H) | 1.02 (d6;3H) |
| 4.46 (t7;1H) | 1.01 (d6;3H) |
| 3.42 (m;1H) | 0.90 (d7;3H) |
| 1.84 (s;3H) | 0.78 (d7;3H) |
| 1.71 (s;3H) | |

## Example 3

(a) A spore suspension (0.4ml) of Streptomyces avermitilis ATCC 31272 [prepared as described for Streptomyces thermoarchaensis NCIB 12015 in Example 1] was used to inoculate a 250ml Erlenmeyer flask containing Medium B (25ml). This culture was incubated at 31°C for 4 days on a rotary shaker operating at 250 rev/min with a 50mm throw, at which time the compound of formula (I) in which $R^1$ is methyl, $R^2$ is methyl, $R^3$ is isopropyl and A is

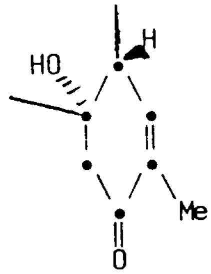

(13mg) in dimethylsulphoxide (0.5ml) was added. The culture was incubated for a further 48h at 31°C, then centrifuged, the supernatant removed and an equal volume of methanol added to the residue. The resulting suspension was allowed to stand for 2h and then centrifuged, the supernatant was evaporated and an equal volume of acetonitrile added to the residue. The solution was evaporated and the residue taken into a small volume of methanol, then subjected to hplc. Comparison with an hplc run using authentic compounds of formula (II) showed the presence in the test sample of compounds of formula (II) (1) where $R^1$ is an isopropyl group and $R^2$ is a hydrogen atom [6% - expressed as % of the starting material added that was converted to the product] and (2) where $R^1$ is an isopropyl group and $R^2$ is a methyl group.

(b) In a similar manner to part (a) the compound of formula (I) in which $R^1$ is methyl, $R^2$ is methyl, $R^3$ is isopropyl and -A- is

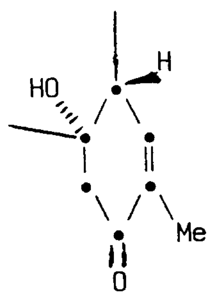

was incubated with (1) Streptomyces thermoarchaensis NCIB 12213 and (2) Streptomyces hygroscopicus subsp. aureolacrimosus FERM P 1438. In each case the culture was prepared from a spore suspension added to Medium B as in part (a), but which after 3 days incubation as described had been used to inoculate fresh Medium B. The compound of formula (I) was added 2 days later to this culture and incubation continued for a further 2 days before the cells were harvested by centrifugation. Methanol extracts of the harvested cells from each culture were analysed by hplc with results as follows [percentages are expressed as % of the starting material added that was converted to the product - comparison with hplc of authentic samples as in part (a)]:

Culture (1) yielded the compound of formula (II) [18.5%] in which $R^1$ is an isopropyl group and $R^2$ is a hydrogen atom.

Culture (2) also yielded the compound of formula (II) [3.1%] in which $R^1$ is an isopropyl group and $R^2$ is a hydrogen atom.

Example 4

(a) A spore suspension (0.1ml) of Streptomyces thermoarchaensis NCIB 12213 [prepared as described for Streptomyces thermoarchaensis 12212 in Example 2] was used to inoculate a flask containing Medium B (2.5ml). This culture was incubated at 31°C for 4 days on a rotary shaker operating at 250 rev/min, at which time the compound of formula (I) in which $R^1$ is methyl, $R^2$ is methyl, $R^3$ is methyl and-A-is

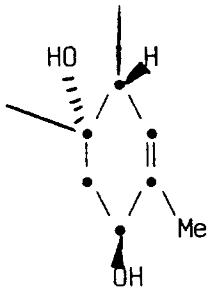

in dimethylsulphoxide (0.05ml) was added. The culture was incubated for a further 24h at 31 ° C, then centrifuged (approx. 10000 rpm/10 min), the supernatant removed and (1.8ml) of methanol added to the residue. The resulting suspension was allowed to stand for 1h at room temperature and then centrifuged. The supernatant was analysed by hplc which showed the presence in the test sample of the compound of formula (II) where $R^1$ is a methyl group and $R^2$ is a hydrogen atom [6.3% - expressed as % of the starting material added that was converted to the product].

(b) In a similar manner to part (a) the compound of formula (I) in which $R^1$ is methyl, $R^2$ is methyl, $R^3$ is methyl and-A-is

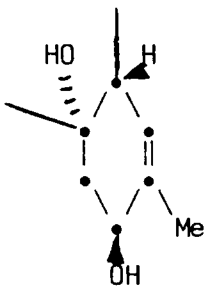

was incubated with Streptomyces thermoarchaensis NCIB 12213, except that the culture was prepared from a spore suspension added to Medium B as in part (a), but which after 3 days incubation as described had been used (0.1ml transferred) to inoculate fresh Medium B. The compound of formula (I) (1mg in 0.05ml dimethylsulphoxide) was added 2 days after to this later culture which was then incubated for a further two days before being harvested, extracted and analysed as desribed in part (a). Hplc analysis of a test sample showed the presence of compounds of formula (II) in which $R^1$ is a methyl group and $R^2$ is a hydrogen atom [4.7% - % expressed as in part (a)] and $R^1$ is a methyl group and $R^2$ is a methyl group [4.1% - % expressed as in part (a)].

(c) A repeat of the part (b) experiment with the same microorganism but the compound of formula (I) in which $R^1$ is methyl, $R^2$ is methyl, $R^3$ is isopropyl and -A- is

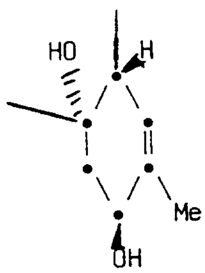

yielded an extract which on hplc analysis showed the presence of the compound of formula (II) in which $R^1$ is an isopropyl group and $R^2$ is a hydrogen atom [1.25% - % expressed as in part (a)].

Example 5

0.4ml of a spore suspension of organism Streptomyces thermoarchaensis NCIB 12015 in 10% glycerol (prepared in a similar manner to the spore suspension used in Example 1) was used to inoculate a 250ml Erlenmyer flask containing Medium A (50ml).

The flask was incubated at 28°C for 2 days on a rotary shaker operating at 250rpm with a 50mm diameter orbital motion. 4ml portions were then used to inoculate each of four 2 litre flat-bottomed flasks, each containing Medium A (200ml) before incubation under the same conditions for 2 days. The contents of the 4 flasks were then used to inoculate a fermenter vessel (70L) containing Medium A (40L) supplemented with polypropylene glycol 2000 (0.06% v/v). Polypropylene glycol 2000 was added as required throughout the fermentation to control foaming. The fermentation was carried out at 28°C, with agitation and aeration sufficient to maintain a dissolved oxygen level of greater than 30% of saturation. After 24 hours of fermentation, 800ml and 9 litre portions were transferred to a fermenter (70L) containing medium (40L), and a fermenter (700L) containing medium (450L), respectively. Both these fermenters contained the following medium:

| | $gL^{-1}$ |
|---|---|
| D-Glucose | 2.5 |
| Malt Dextrin (MD3OE) | 25.0 |
| Arkasoy 50 | 12.5 |
| Beet Molasses | 1.5 |
| $K_2HPO_4$ | 0.125 |
| $CaCO_3$ | 1.25 |
| Silicone 1520 (Dow Corning) | 0.6 |

Distilled water to 1 litre. pH was adjusted to 6.5 with aqueous $H_2SO_4$ before sterilisation.

These fermentations were carried out at 34°C with agitation and aeration sufficient to maintain a dissolved oxygen level of greater than 30% of saturation. Polypropylene glycol 2000 antifoam was added as required. The pH in each fermenter was controlled down to 7.2 with the addition of aqueous $H_2SO_4$. The fermentations were harvested and bulked after 4 days.

The mycelium (10.4kg) from the bulked harvest broth (423L) was collected in a centrifuge. The mycelium was stirred vigorously in methanol (50L) for 40 min and then filtered. The residue was resuspended in methanol (15L) and again filtered. The combined filtrates (55L) were then mixed with water (27L) and PE (30L) and stirred for 20 min. The phases were separated in a centrifuge, and the methanol phase (75L) was mixed with more water (38L) and PE (30L). After 20 min the phases were again separated in the centrifuge, acetone (2L) being added to the PE phase to break the emulsion. The methanol phase (110L) was mixed with water (38L) and PE (30L) for a third time, the phases being separated as before. Acetone (3L) was again added to the PE phase to break the emulsion. The three PE phases were bulked (90L) and concentrated at low pressure. The concentrate (9.8L) was dried with sodium sulphate (3Kg) and then evaporated to an oil (560g).

The oil was dissolved in dichloromethane (0.5L) and filtered through Dicalite 478. The solution (0.9L) was subjected to CCM [150 x 10cm column packed and washed with dichloromethane (4L)]. The fraction eluting between 14.6 and 33.3L was concentrated to a solid (170g) and redissolved in $CHCl_3$:EA (3:1).

The solution was rechromatographed using the same system. The fraction eluting between 14.5 and 31.5L was dried to a solid (164g) and dissolved in $CHCl_3$:EA (3:1). This solution was again chromatographed on silica under the same conditions as before and the fraction eluting between 14 and 31L was dried to a solid (123g).

The solid was dissolved in 70% acetonitrile in water (1.23L) with enough methanol added to give a clear solution. The solution was chromatographed in 5ml portions on a column (250 x 20mm) of Spherisorb ODS2 (5$\mu$ particle size). The column was eluted with 70% acetonitrile at a flow rate which increased from 20mL/min to 34mL/min over 21 min. The fractions from each portion which eluted between 12.4 and 16 min were collected together and diluted with an equal volume of water. This solution was then loaded onto a column of Montedison S112 macroreticular polystyrene (2L). The column was washed with 35% acetonitrile and eluted with acetone. The fraction eluting between 0.5 and 1.25L was dried to a solid. The solid was dissolved in acetonitrile (20mL) and chromatographed on a column (110 x 4.4cm) of Sephadex LH20 in the same solvent.

The fractions eluting between 1.08L and 1.26L were collected and dried to a solid (2.0g). The solid was dissolved in 60% acetonitrile (20ml) with enough methanol added to give a clear solution. It was chromatographed in 2ml portions on a column (250 x 20mm) of Spherisorb S5 ODS2 and eluted with 60% acetonitrile at 25ml/min. The fractions eluting between 0.58L and 0.85L were combined and evaporated to a solid (0.87g). The solid was dissolved in $CHCl_3$/EA (9:1, 5ml) and fractionated (21ml) on a column (35x2.2cm) of Kieselgel 60 (Merck, article no. 7734) in the same solvent mixture. Combination of fractions 17-22 followed by evaporation yielded a colourless solid (120mg). The solid was the compound of formula (I) in which $R^1$ is methyl, $R^2$ is hydroxymethyl, $R^3$ is methyl and-A-is

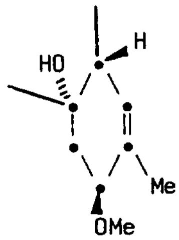

and had the following characteristics.

$^1$H NMR SPECTRUM (ca 2% $CDCl_3$ solution)

A 200MHz $^1$H NMR spectrum has (among others) signals centred at about

| | |
|---|---|
| δ 0.78 (d7;3H)<br>1.01 (1d6;3H)<br>1.57 (s;3H)<br>1.59 (s;3H)<br>1.63 (d7;3H)<br>1.80 (s;3H)<br>3.35 (s;3H) | 4.16 (dd14,4;1H)<br>4.27 (dd14,6;1H)<br>5.28 (narrow m;1H)<br>6.25 (dd16,11;1H)<br>6.38 (d11;1H) |

$^{13}$C NMR SPECTRUM ($CDCl_3$ solution)

A 62.6MHz $^{13}$C NMR spectrum includes signals at about the following positions (multiplicities due to proton coupling in parentheses).

| | |
|---|---|
| δ 173.2(s) | 57.7(t) |
| 143.6(d) | 56.4(q) |
| 139.6(s) | 48.9(d) |
| 138.3(s) | 48.2(t) |
| 136.6(s) | 40.9(t) |
| 133.7(s) | 40.5(t) |
| 129.3(d) | 37.3(t) |
| 123.9(d) | 36.1(?) |
| 123.7(d) | 36.0(?) |
| 120.0(d) | 35.8(?) |
| 118.2(d) | 34.4(t) |
| 99.6(s) | 21.5(q) |
| 76.6(d) | 19.0(q) |
| 76.5(d) | 15.7(q) |
| 75.6(s) | 13.6(q) |
| 69.0(d) | 12.8(q) |
| 68.4(d) | 10.5(q) |
| 67.7(d) | |

The multiplicities of the close signals at δ36.1, 36.0 and 35.8 could not be reliably established.

<u>I.R. SPECTRUM</u> (bromoform solution)

shows absorption bands at about 3440 (OH), 1704 ($CO_2R$) and 992cm$^{-1}$ (C-O).

<u>U.V. SPECTRUM</u>

The spectrum of a ca 0.002% solution in methanol shows bands at 242nm (max) $E_1^1$ 444, 250nm (inf) $E_1^1$ 323, 279nm (max) $E_1^1$ 26.

<u>MASS SPECTRUM</u>

An F.D. mass spectrum (AM823) gave a molecular ion at 600 nominal mass.

<u>Example 6</u>

0.4 ml of a spore suspension of organism <u>Streptomyces thermoarchaensis</u> NC1B 12015 in 10% glycerol (prepared in a similar manner to the spore suspension used in Example 1) was used to inoculate a 250ml Erlenmyer flask containing Medium B (50ml).

The flask was incubated at 28°C for 2 days on a rotary shaker operated at 250 rpm with a 50mm diameter orbital motion. 2% (v/v) portions of this 2 day developed culture was used to inoculate further 250 ml Erlenmyer flasks containing Medium B (50ml) before incubation under the same conditions for 2 days. 80ml of the combined contents was used to inoculate a fermenter vessel (7L) containing the following medium.

| | gL$^{-1}$ |
|---|---|
| D-glucose | 4.4 |
| Malt Dextrin (MD3OE) | 44.4 |
| Arkasoy 50 | 22.0 |
| Molasses | 2.6 |
| $K_2HPO_4$ | 0.21 |
| Calcium carbonate | 2.1 |
| Silicone 1520 (Dow Corning) | 2.5 |

Distilled water to 1.75 L, pH adjusted to 6.5 with aqueous $H_2SO_4$ before sterilisation.

The fermentation was carried out at 34°C with agitation and aeration. The pH was controlled down to 7.4 with the addition of propionic acid. The fermentation was harvested after 138h.

The mycelium from the harvest broth (3L) was collected in a centrifuge. The mycelium was suspended in water (1L) and again centrifuged. The supernatant was discarded and the cells were suspended in methanol (500ml) and centrifuged. The methanol extract was evaporated to give an oil which was extracted with acetonitrile (150ml). A portion (30ml) of the extract was fractionated (17ml) on a column (32 x 2.2cm) of Sephadex LH20 in acetone. Fractions 5-16 were combined and evaporated to yield a solid (507mg).

The solid was examined by H.P.L.C. on a column (250 x 2mm) of Aldex ODS2 in a 3:1 acetonitrile/water solvent system at a flow rate of 0.2ml/min. After 16½ min. the column effluent was interfaced with a Finnigan moving belt to a Finnigan MAT 4500 mass spectrometer under E.I. conditions and the mass spectra of materials were recorded. Compounds identified include the compound of formula (I) in which $R^1$ is methyl, $R^2$ is methyl, $R^3$ is ethyl and -A- is

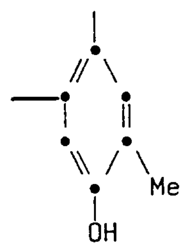

(low resolution E.I. spectrum gave fragment ions at m/z 546, 528, 408, 251, 245, 233, 227, 223 and 205).

Example 7

1.0 ml of a spore suspension of organism Streptomyces thermoarchaensis NCIB 12334 in 20% glycerol (prepared in a similar manner to the spore suspension in Example 1) was used to inoculate a 250 Erlenmyer flask containing Medium B (50 ml).

The flask was incubated at 28° for 2 days at which time 1ml was asepticially transferred into each of four similar flasks (each containing 50ml Medium B). Each flask was incubated at 28° for a 2 day period with agitation on a rotary shaker (250 rev/min) with a 50mm diameter throw. Two 7L seed fermenters were then inoculated with about 80ml [2% (v/v)] of the bulked shake flask inoculum. Each 7L fermenter contained 4L of Medium C:

Medium C

| | g/L |
|---|---|
| Meritose | 45.0 |
| Arkasoy 50 | 18.0 |
| Beet Molasses | 2.2 |
| $K_2HPO_4$ | 0.18 |
| $CaCO_3$ (Analar) | 1.8 |
| Silcone 1520 (Dow Corning) | 2.5ml in 4L |

Distilled water to 4L with pH adjusted to 6.5 prior to autoclaving (45 min at 124°). The fermentation was carried out at 28° with agitation and aeration.

A 700L fermenter was inoculated with 5.4 litres transferred aseptically from the seed vessels at 24h. The fermenter contained 450L of Medium C in which $CaCO_3$ (Analar) was replaced by Sturcal chalk and 250ml of silicone 1520 was batched in. The pH was adjusted to 6.5 using concentrated $H_2SO_4$ prior to in situ sterilization for 30 min at 121°. In addition three 70L fermenters were inoculated with 800ml (2% v/v) transferred aseptically from the seed vessel at 24h. Each vessel contained 35L of Medium C as shown above with 25ml of silicone 1520 added prior to in situ sterilization.

The subsequent fermentations were carried out at 34° with agitation and aeration. Polypropylene glycol 2000 antifoam was added as required throughout the fermentations.

After 120h the contents of the fermenters were bulked (600L), mixed with Dicalite (3kg) and filtered through cellulose (24kg Rettenmaier BEOO) on a rotary vacuum filter. The recovered cell paste (45kg) was stored at -20°.

The frozen cell paste was suspended in methanol (55L) and allowed to thaw. After 30 min at 5° the suspension was filtered through a cellulose bed. The residue was resuspended in methanol (50L), filtered through the cellulose bed and washed with methanol (10L).

The combined filtrates and washes (116L) were diluted with water (22L) and extracted with 60°-80° petroleum ether (100L). The hexane extract was concentrated at low pressure (approx. 0.15 bar) in a wiped-film evaporated.

The concentrate (8.4L) was extracted three times with propan-1,2-diol (3x4.2L). The combined extracts (13.5L) were stirred with ethyl acetate (22L) and water (12L), and were then left to settle. The upper phase (17.5L) was washed twice with water (12L and 10L) and the remaining organic phase was dried to an oil (112g).

The oil was dissolved in dichloromethane (500mL) and chromatographed on a column (425mm x 45mm diam) of Merck Kieselgel 60 (type 7734) packed in the same solvent. The column was washed with dichloromethane (500mL) and was then eluted with a mixture of dichloromethane and diethyl ether (400mL of 9:1 v/v respectively). The eluate was dried to a pale yellow tar (54g).

A portion of the tar (4.32g) dissolved in diethyl ether was dried to a solid, and redissolved in acetonitrile at a concentration of 300mg/ml. This solution (8.5ml) was mixed with an equal volume of 75% v/v acetonitrile in water and was chromatographed in nine portions on a column (250mm x 20mm) of Spherisorb ODS2. The several components were eluted (25ml/min) using 75% acetonitrile.

The peak eluting between 0.42L and 0.46L was collected from each run and bulked. The bulk was mixed with an equal volume of water and was readsorbed on the same column. The column was eluted with acetonitrile, the eluate warmed to 45°C and water added until the solution was just cloudy. This was cooled to 4°C and left to crystallise. A sample (50mg) of the dried crystals (80mg) was dissolved in a 10ml mixture of hexane/methylene chloride/methanol (200:5:3) and chromatographed in 5 portions on a column (250 x 20mm) of silica using the same solvent system as eluant and at a flow rate of 20ml/min. Two parts were collected and separately bulked from each run. The first, which eluted between 0.37 and 0.4L was concentrated and dried from a mixture of acetone and cyclohexane to give 23mg of the compound of formula (I) in which $R^1$ is methyl, $R^2$ is an aldehydo group, $R^3$ is isopropyl and -A- is

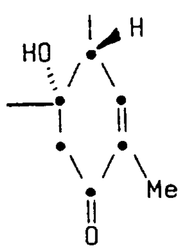

A 500MHz $^1H$ NMR spectrum of a deuterochloroform solution includes signals at about

| | |
|---|---|
| δ 0.79 (d7;3H) | 3.67 (m;1H) |
| 0.94 (d7;3H) | 3.71 (d11;1H) |
| 1.02 (d7;3H) | 5.84 (dd 10,15; 1H) |
| 1.10 (d7;3H) | 6.43 (m;1H) |
| 1.57 (d2;3H) | 6.80 (dd 12, 15;1H) |
| 1.61 (s;3H) | 7.38 (d12;1H) |
| 1.86 (dd 2, 3;3H) | 10.22 (s;1H) |
| 2.39 (d17;1H) | |
| 3.38 (dd 17, 2;1H) | |

A 125$MH_z$ $^{13}C$ NMR spectrum of a deuterochloroform solution has signals at about

| | | |
|---|---|---|
| 196.2 (s) | 76.5 (d) | 26.6 (d) |
| 189.0 (d) | 75.6 (s) | 22.6 (q) |
| 172.7 (s) | 69.0 (d) | 22.5 (q) |
| 153.3 (d) | 68.1 (d) | 21.0 (q) |
| 147.9 (d) | 68.0 (d) | 15.9 (q) |
| 137.1 (d) | 48.4 (d) | 15.4 (q) |
| 136.4 (s) | 47.7 (t) | 13.6 (q) |
| 136.2 (s) | 47.6 (t) | 10.7 (q) |
| 136.1 (d) | 40.9 (t) | |
| 135.6 (s) | 40.2 (t) | |
| 130.3 (s) | 36.5 (d) | |
| 121.2 (d) | 35.9 (t) | |
| 120.6 (d) | 35.8 (d) | |
| 99.5 (s) | 34.3 (t) | |

The following are examples of formulations according to the invention. The term 'Active Ingredient' as used hereinafter means a compound of the invention.

Multidose parenteral injection

| | % w/v | Range |
|---|---|---|
| Active Ingredient | 4.0 | 0.1 - 7.5% w/v |
| Benzyl alcohol | 2.0 | |
| Glyceryl triacetate | 30.0 | |
| Propylene glycol | to 100.0 | |

Dissolve the active ingredient in the benzyl alcohol and glyceryl triacetate. Add propylene glycol and make up to volume. Sterilise the product by conventional pharmaceutical methods, for example sterile filtration or by heating in an autoclave and package aseptically.

Aerosol spray

| | % w/w | Range |
|---|---|---|
| Active Ingredient | 0.1 | 0.01 - 2.0% w/w |
| Trichloroethane | 29.9 | |
| Trichlorofluoromethane | 35.0 | |
| Dichlorodifluoromethane | 35.0 | |

Mix the Active Ingredient with trichloroethane and fill into the aerosol container. Purge the headspace with the gaseous propellant and crimp the valve into position. Fill the required weight of liquid propellant under pressure through the valve. Fit with actuators and dust-caps.

Tablet

Method of manufacture - wet granulation

| | mg |
|---|---|
| Active Ingredient | 250.0 |
| Magnesium stearate | 4.5 |
| Maize starch | 22.5 |
| Sodium starch glycolate | 9.0 |
| Sodium lauryl sulphate | 4.5 |

Microcrystalline cellulose to tablet core weight of 450mg Add sufficient quantity of a 10% starch paste to the active ingredient to produce a suitable wet mass for granulation. Prepare the granules and dry using a tray or fluid-bed drier. Sift through a seive, add the remaining ingredients and compress into tablets.

If required, film coat the tablet cores using hydroxypropylmethyl cellulose or other similar film-forming material using either an aqueous or non-aqueous solvent system. A plasticizer and suitable colour may be included in the film-coating solution.

Veterinary tablet for small/domestic animal use

Method of manufacture - dry granulation

| | mg |
|---|---|
| Active Ingredient | 50.0 |
| Magnesium stearate | 7.5 |
| Microcrystalline cellulose to tablet core weight of | 75.0 |

Blend the active ingredient with the magnesium stearate and microcrystallise cellulose. Compact the blend into slugs. Break down the slugs by passing through a rotary granulator to produce free-flowing granules. Compress into tablets.

The tablet cores can then be film-coated, if desired, as described above.

Veterinary intrammary injection

| | mg/dose | Range |
|---|---|---|
| Active Ingredient | 150mg | 0.05 - 1.0g |
| Polysorbate 60 | 3.0% w/w) ) | |
| White Beeswax | 6.0% w/w) to 3g ) | to 3 or 15g |
| Arachis oil | 91.0% w/w) ) | |

Heat the arachis oil, white beeswax and polysorbate 60 to 160 °C with stirring. Maintain at 160 °C for two hours and then cool to room temperature with stirring. Aseptically add the active ingredient to the vehicle and disperse using a high speed mixer. Refine by passing through a colloid mill. Aseptically fill the product into sterile plastic syringes.

Veterinary oral drench

| | % w/v | Range |
|---|---|---|
| Active Ingredient | 0.35 | 0.01 - 2% w/v |
| Polysorbate 85 | 5.0 | |
| Benzyl alcohol | 3.0 | |
| Propylene glycol | 30.0 | |
| Phosphate buffer | as pH 6.0 - 6.5 | |
| Water | to 100.0 | |

Dissolve the active ingredient in the Polysorbate 85, benzyl alcohol and the propylene glycol. Add a proportion of the water and adjust the pH to 6.0 - 6.5 with phosphate buffer, if necessary. Make up to final volume with the water. Fill the product into the drench container.

Veterinary oral paste

| | % w/w | Range |
|---|---|---|
| Active Ingredient | 7.5 | 1 - 30% w/w |
| Saccharin | 25.0 | |
| Polysorbate 85 | 3.0 | |
| Aluminium distearate | 5.0 | |
| Fractionated coconut oil | to 100.0 | |

Disperse the aluminium distearate in the fractionated coconut oil and polysorbate 85 by heating. Cool to room temperature and disperse the saccharin in the oily vehicle. Dispense the active ingredient in the base. Fill into plastic syringes.

Granules for veterinary in-feed administration

| | % w/w | Range |
|---|---|---|
| Active Ingredient | 2.5 | 0.05-5% w/w |
| Calcium sulphate, hemi-hydrate | to 100.0 | |

Blend the Active Ingredient with the calcium sulphate. Prepare the granules using a wet granulation process. Dry using a tray or fluid-bed drier. Fill into the appropriate container.

Emulsifiable Concentrate

| | |
|---|---|
| Active ingredient | 50g |
| Anionic emulsifier (e.g. Phenyl sulphonate CALX) | 40g |
| Non-ionic emulsifier (e.g. Syperonic NP13) | 60g |

Aromatic solvent (e.g. Solvesso 100) to 1 litre.
Mix all ingredients, stir until dissolved.

Granules

| | | |
|---|---|---|
| (a) | Active ingredient<br>Wood resin | 50g<br>40g |
| Gypsum granules (20-60 mesh) to 1kg (e.g. Agsorb 100A) | | |

| | | |
|---|---|---|
| (b) | Active ingredient<br>Syperonic NP13 | 50g<br>40g |
| Gypsum granules (20-60 mesh) to 1kg. | | |

Dissolve all ingredients in a volatile solvent e.g. methylene chloride, add to granules tumbling in mixer. Dry to remove solvent.

**Claims**

**Claims for the following Contracting States : BE, CH, DE, FR, GB, IT, LI, NL, SE**

**1.** Compounds having the formula (I)

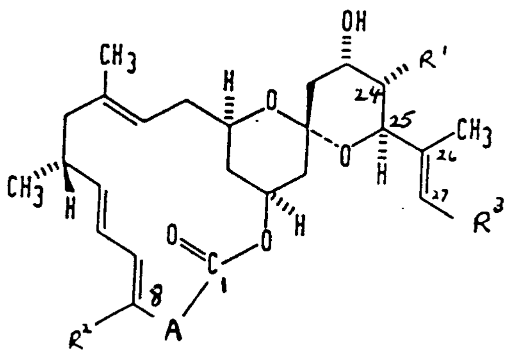

(I)

wherein

$R^1$ represents a methyl group;

$R^2$ represents a methyl group;

$R^3$ represents a methyl, ethyl or isopropyl group; and

-A- represents a ring which is either

(i) 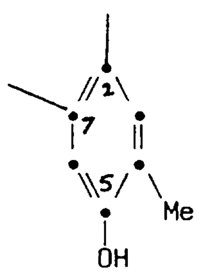 or (ii) 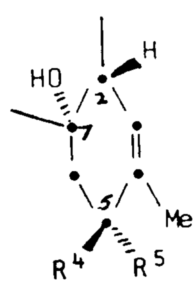

(where $R^4$ is a hydroxy or methoxy group and $R^5$ is a hydrogen atom or $R^4$ and $R^5$ together with the carbon atom to which they are attached represent a group C=0); or when -A- represents ring (ii) wherein $R^4$ and $R^5$ together with the carbon atom to which they are attached represent a group C=0 then $R^2$ may also represent an aldehydo group;

or $R^2$ represents a hydroxymethyl group and -A- represents

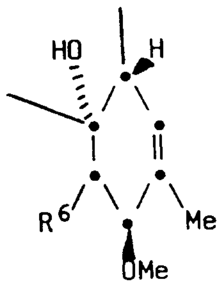

(where $R^6$ represents a hydrogen atom or a hydroxy group);

with the provisos that when $R^2$ represents a methyl group and $R^4$ represents a methoxy group then $R^3$ cannot represent a methyl or isopropyl group and when $R^2$ represents a hydroxymethyl group and $R^6$ represents a hydroxy group then $R^3$ cannot represent a methyl group.

**2.** Compounds according to claim 1 of formula (I) in which $R^3$ is an isopropyl group.

**3.** Compounds according to claim 1 of formula (I) in which $R^1$ and $R^2$ are methyl groups, $R^3$ is an isopropyl group and -A- is a group (ii) in which $R^4$ is a hydroxy group and $R^5$ is a hydrogen atom or $R^4$ and $R^5$ together with the carbon atom to which they are attached represent C=O.

**4.** A composition for use in human medicine containing an effective amount of at least one compound according to claim 1 together with one or more carriers and/or excipients.

**5.** A composition for use in veterinary medicine containing an effective amount of at least one compound according to claim 1 together with one or more carriers and/or excipients.

**6.** A pest control composition containing an effective amount of at least one compound according to claim 1 together with one or more carriers and/or excipients.

**7.** A method for combatting pests in agriculture, horticulture or forestry, or in stores, buildings or other public places or locations of the pests, which comprises applying to plants or other vegetation or to the pests themselves or a location thereof an effective amount of one or more compounds according to claim 1.

**8.** A method as claimed in claim 7 in which said pests are insect, acarine or nematode pests.

**9.** A process for the preparation of a compound according to claim 1 which comprises the step of cultivating an organism of the genus Streptomyces capable of producing at least one said compound whereby at least one of said compounds is produced, and if desired isolating said compound therefrom.

**10.** A process according to claim 9 in which the organism of the genus Streptomyces is the species Streptomyces thermoarchaensis or Streptomyces cyaneogriseus noncyanogenus.

**11.** A process according to claim 9 in which said organism is Streptomyces thermoarchaensis NCIB 12015 NCIB 12111, NCIB 12112, NCIB 12113, NCIB 12114, NCIB 12212 or NCIB 12334, or Streptomyces cyaneogriseus noncyanogenus NRRL 15773 or a mutant of any of these strains.

**12.** Streptomyces thermoarchaensis NCIB 12212 and its mutants and the genetic material thereof that participates in the synthesis of compounds according to claim 1.

**13.** The use of a compound of formula (I) according to claim 1 in the preparation of a compound of formula (II) below (or a 5-keto derivative thereof)

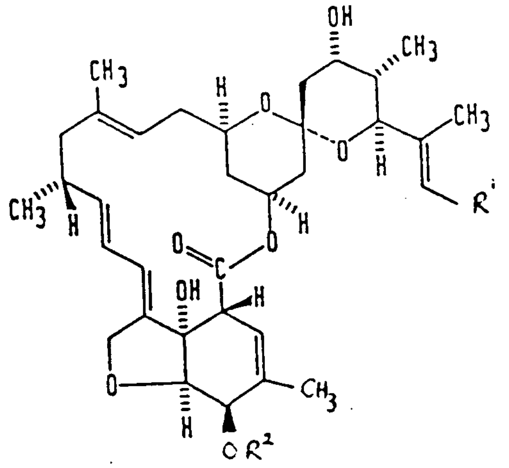

(II)

(wherein $R^1$ is a methyl, ethyl or isopropyl group and $R^2$ is a hydrogen atom or a methyl group) which comprises cultivating a suitable Streptomyces organism in the presence of a said compound of formula (I), whereby a compound of formula (II), is produced, and if desired isolating the said compound.

**14.** The use according to claim 13 of a compound according to claim 3 in the production of a compound of formula (II) in which $R^1$ is an isopropyl group and $R^2$ is a hydrogen atom.

**15.** Use according to claim 13 or claim 14 in which the organism is a strain of Streptomyces thermoarchaensis, Streptomyces cyaneogriseus noncyanogenus, Streptomyces avermitilis or Streptomyces hygroscopicus subsp. aureolacrimosus.

**16.** Use according to claim 15 in which the organism is Streptomyces thermoarchaensis NCIB 12213, or a mutant thereof, Streptomyces avermitilis ATCC 31272 or Streptomyces hygroscopicus subsp. aureolacrimosus FERM P 1438.

**17.** Streptomyces thermoarchaensis NCIB 12213 and mutants thereof, and the genetic material thereof that participates in the synthesis of compounds of formula (II) as defined in claim 13.

**Claims for the following Contracting States : AT, ES**

**1.** A process for the preparation of compounds having the formula (I)

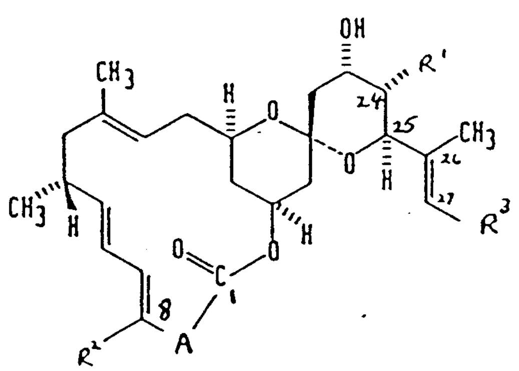

(I)

wherein

$R^1$ represents a methyl group;
$R^2$ represents a methyl group;
$R^3$ represents a methyl, ethyl or isopropyl group; and
-A- represents a ring which is either

(i) 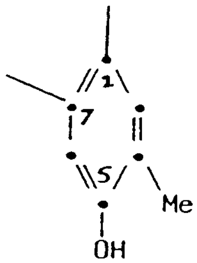 or (ii) 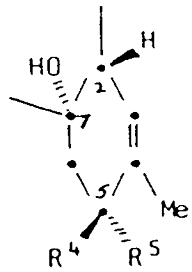

(where $R^4$ is a hydroxy or methoxy group and $R^5$ is a hydrogen atom or $R^4$ and $R^5$ together with the carbon atom to which they are attached represent a group C=O); or when -A- represents ring (ii) wherein $R^4$ and $R^5$ together with the carbon atom to which they are attached represent a group C=O then $R^2$ may also represent an aldehydo group;

or $R^2$ represents a hydroxymethyl group and -A-represents

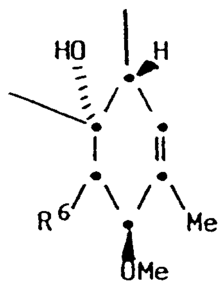

(where $R^6$ represents a hydrogen atom or a hydroxy group); with the provisos that when $R^2$ represents a methyl group and $R^4$ represents a methoxy group then $R^3$ cannot represent a methyl or isopropyl group and when $R^2$ represents a hydroxymethyl group and $R^6$ represents a hydroxy group then $R^3$ cannot represent a methyl group,

which process comprises the step of cultivating an organism of the genus Streptomyces capable of producing at least one said compound whereby at least one of said compounds is produced, and if desired isolating said compound therefrom.

**2.** A process according to claim 1 in which $R^3$ in the product is an isopropyl group.

**3.** A process according to claim 1 in which, in the product, $R^1$ and $R^2$ are methyl groups, $R^3$ is an isopropyl group and -A- is a group (ii) in which $R^4$ is a hydroxy group and $R^5$ is a hydrogen atom or $R^4$ and $R^5$ together with the carbon atom to which they are attached represent C=O.

**4.** A process according to any preceding claim in which the organism of the genus Streptomyces is the species Streptomyces thermoarchaensis or Streptomyces cyaneogriseus noncyanogenus.

**5.** A process according to claim 4 in which said organism is NCIB 12015, NCIB 12111, NCIB 12112, NCIB 12113, NCIB 12114, NCIB 12212 or NCIB 12334, or Streptomyces cyaneogriseus noncyanogenus NRRL 15773 or a mutant of any of these strains.

**6.** A pest control composition (for use other than as a pharmaceutical composition) containing an effective amount of at least one compound of formula (I) as defined in claim 1 together with one or more carriers and/or excipients.

**7.** A method for combatting pests in agriculture, horticulture or forestry, or in stores, buildings or other public places or locations of the pests, which comprises applying to plants or other vegetation or to the pests themselves or a location thereof an effective amount of one or more compounds of formula (I) as defined in claim 1.

**8.** A method as claimed in claim 7 in which said pests are insect, acarine or nematode pests.

**9.** Streptomyces thermoarchaensis NCIB 12212 and its mutants and the genetic material thereof that participates in the synthesis of compounds according to claim 1.

**10.** The use of a compound of formula (I) as defined in claim 1 in the preparation of a compound of formula (II) below (or a 5-keto derivative thereof)

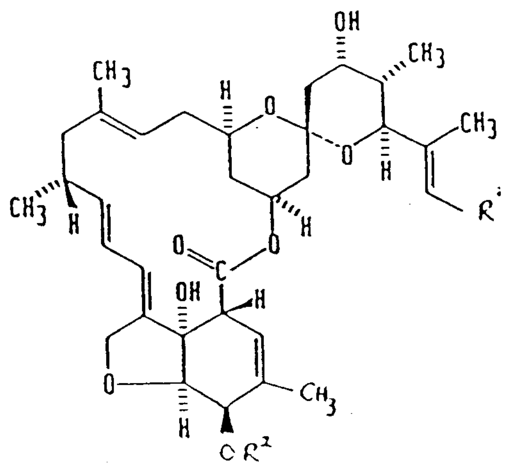

(II)

(where $R^1$ is a methyl, ethyl or isopropyl group and $R^2$ is a hydrogen atom or a methyl group) which comprises cultivating a suitable Streptomyces organism in the presence of a said compound of formula (I), whereby a compound of formula (II) is produced, and if desired isolating the said compound.

**11.** The use according to claim 10 of a compound as defined in claim 3 in the production of a compound of formula (II) in which $R^1$ is an isopropyl group and $R^2$ is a hydrogen atom.

**12.** Use according to claim 10 or claim 11 in which the organism is a strain of Streptomyces thermoarchaensis, Streptomyces cyaneogriseus noncyanogenus, Streptomyces avermitilis or Streptomyces hygroscopicus subsp. aureolacrimosus.

**13.** Use according to claim 12 in which the organism is Streptomyces thermoarchaensis NCIB 12213, or a mutant thereof, Streptomyces avermitilis ATCC 31272 or Streptomyces hygroscopicus subsp. aureolacrimosus FERM. P1438.

**14.** Streptomyces thermoarchaensis NCIB 12213 and mutants thereof, and the genetic material thereof that participates in the synthesis of compounds of formula (II) as defined in claim 10.

**Patentansprüche**
**Patentansprüche für folgende Vertragsstaaten : BE, CH, DE, FR, GB, IT, LI, NL, SE**

**1.** Verbindungen der Formel (I)

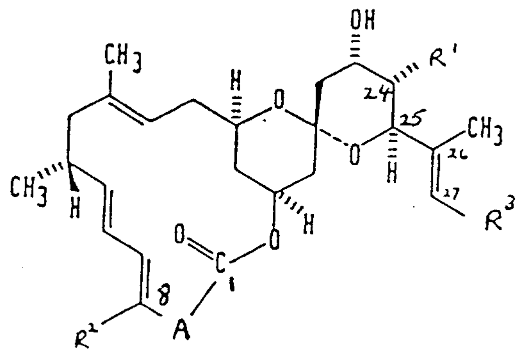

(I)

worin
$R^1$ für eine Methylgruppe steht;
$R^2$ für eine Methylgruppe steht;
$R^3$ für eine Methyl-, Ethyl- oder Isopropylgruppe steht; und
-A- einen Ring wiedergibt, der entweder

(i) 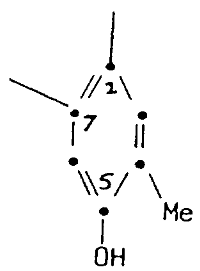 oder (ii) 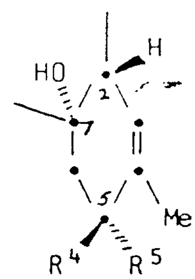

ist (worin $R^4$ für eine Hydroxy- oder Methoxygruppe steht und $R^5$ ein Wasserstoffatom bedeutet oder $R^4$ und $R^5$ gemeinsam mit den Kohlenstoffatom, an das sie gebunden sind, eine Gruppe C=O wiedergeben); oder, wenn -A- den Ring (ii) wiedergibt, worin $R^4$ und $R^5$ gemeinsam mit dem Kohlenstoffatom, an das sie gebunden sind, eine Gruppe C=O bilden, $R^2$ auch eine Aldehydgruppe bedeuten kann;
oder $R^2$ eine Hydroxymethylgruppe wiedergibt und -A-

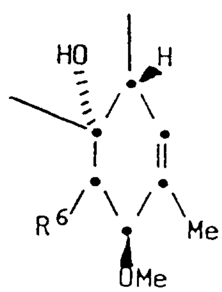

(worin $R^6$ ein Wasserstoffatom oder eine Hydroxygruppe bedeutet) darstellt; mit den Maßgaben, daß, wenn $R^2$ für eine Methylgruppe steht und $R^4$ für eine Methoxygruppe steht, $R^3$ nicht eine Methyl- oder Isopropylgruppe bedeuten kann, und wenn $R^2$ für eine Hydroxymethylgruppe steht und $R^6$ für eine Hydroxygruppe steht, $R^3$ nicht eine Methylgruppe bedeuten kann.

**2.** Verbindungen gemäß Anspruch 1 der Formel (I), worin $R^3$ eine Isopropylgruppe bedeutet.

**3.** Verbindungen gemäß Anspruch 1 der Formel (I), worin $R^1$ und $R^2$ Methylgruppen bedeuten, $R^3$ eine Isopropylgruppe ist und -A- eine Gruppe (ii) bedeutet, worin $R^4$ für eine Hydroxygruppe steht und $R^5$ ein Wasserstoffatom bedeutet oder $R^4$ und $R^5$ gemeinsam mit dem Kohlenstoffatom, an das sie gebunden sind, für C=O stehen.

**4.** Zusammensetzung für die Verwendung in der Humanmedizin, enthaltend eine wirksame Menge zumindest einer Verbindung gemäß Anspruch 1 zusammen mit einem oder mehreren Trägern und/oder Exzipienten.

**5.** Zusammensetzung für die Verwendung in der Veterinärmedizin, enthaltend eine wirksame Menge zumindest einer Verbindung gemäß Anspruch 1 zusammen mit einem oder mehreren Trägern und/oder Exzipienten.

**6.** Zusammensetzung für die Schädlingskontrolle, enthaltend eine wirksame Menge zumindest einer Verbindung gemäß Anspruch 1 zusammen mit einem oder mehreren Trägern und/oder Exzipienten.

**7.** Verfahren zur Bekämpfung von Schädlingen in der Landwirtschaft, im Gartenbau oder der Forstwirtschaft oder in Warenhäusern, Gebäuden oder anderen öffentlichen Plätzen oder Aufenthaltsorten der Schädlinge, das das Aufbringen einer wirksamen Menge einer oder mehrerer Verbindungen der Formel (I), wie in Anspruch 1 definiert, auf Pflanzen oder andere Vegetation oder auf die Schädlinge selbst oder deren Aufenthaltsort umfaßt.

**8.** Verfahren gemäß Anspruch 7, worin die Schädlinge Insekten, Milben oder Nematodenschädlinge sind.

**9.** Verfahren zur Herstellung einer Verbindung gemäß Anspruch 1, das die Stufe der Kultivierung eines Organismus der Gattung Streptomyces, der zur Bildung zumindest einer dieser Verbindungen befähigt ist, wodurch zumindest eine dieser Verbindungen gebildet wird, und gewünschtenfalls die Isolierung dieser Verbindung daraus umfaßt.

**10.** Verfahren gemäß Anspruch 9, worin der Organismus der Gattung Streptomyces die Species Streptomyces thermoarchaensis oder Streptomyces cyaneogriseus noncyanogenus ist.

**11.** Verfahren gemäß Anspruch 9, worin der Organismus Streptomyces thermoarchaensis NCIB 12015, NCIB 12111, NCIB 12112, NCIB 12113, NCIB 12114, NCIB 12212 oder NCIB 12334 oder Streptomyces cyaneogriseus noncyanogenus NRRL 15773 oder eine Mutante irgendeines dieser Stämme ist.

**12.** Streptomyces thermoarchaensis NCIB 12212 und dessen Mutanten und dessen genetisches Material, das bei der Synthese der Verbindungen gemäß Anspruch 1 teilhat.

**13.** Verwendung einer Verbindung der Formel (I), wie in Anspruch 1 definiert, bei der Herstellung einer Verbindung der folgenden Formel (II) (oder eines 5-Ketoderivats hiervon)

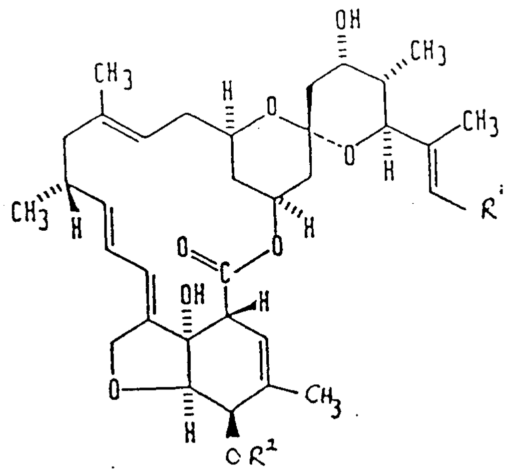

(II)

(worin $R^1$ eine Methyl-, Ethyl- oder Isopropylgruppe bedeutet und $R^2$ für ein Wasserstoffatom oder eine Methylgruppe steht), das die Kultivierung eines geeigneten Streptomyces-Organismus in Anwesenheit der Verbindung der Formel (I), wodurch eine Verbindung der Formel (II) gebildet wird, und gewünschtenfalls die Isolierung dieser Verbindung umfaßt.

**14.** Verwendung gemäß Anspruch 13 einer Verbindung, wie in Anspruch 3 definiert, bei der Herstellung einer Verbindung der Formel (II), worin $R^1$ eine Isopropylgruppe bedeutet und $R^2$ für ein Wasserstoffatom steht.

**15.** Verwendung gemäß Anspruch 13 oder 14, worin der Organismus ein Stamm von Streptomyces thermoarchaensis, Streptomyces cyaneogriseus noncyanogenus, Streptomyces avermitilis oder Streptomyces hygroscopicus subst.aureolacrimosus ist.

**16.** Verwendung gemäß Anspruch 15, worin der Organismus Streptomyces thermoarchaensis NCIB 12213 oder eine Mutante hiervon, Streptomyces avermitilis ATCC 31272 oder Streptomyces hygroscopicus subsp.aureolacrimosus FERM P1438 ist.

**17.** Streptomyces thermoarchaensis NCIB 12213 und dessen Mutanten und dessen genetisches Material, das bei der Synthese der Verbindungen der Formel (II), wie in Anspruch 13 definiert, teilhat.

**Patentansprüche für folgende Vertragsstaaten : AT, ES**

**1.** Verfahren zur Herstellung von Verbindungen der Formel (I)

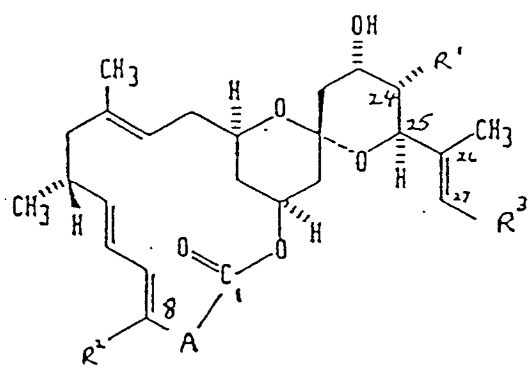

(I)

worin
$R^1$ für eine Methylgruppe steht;
$R^2$ für eine Methylgruppe steht;
$R^3$ für eine Methyl-, Ethyl- oder Isopropylgruppe steht; und

-A- einen Ring wiedergibt, der entweder

(i) 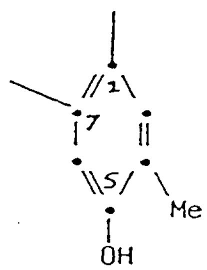 oder (ii) 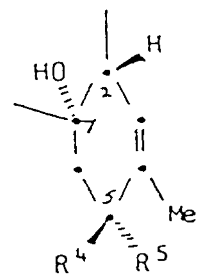

ist (worin $R^4$ für eine Hydroxy- oder Methoxygruppe steht und $R^5$ ein Wasserstoffatom bedeutet oder $R^4$ und $R^5$ gemeinsam mit dem Kohlenstoffatom, an das sie gebunden sind, eine Gruppe C = O wiedergeben); oder, wenn -A- den Ring (ii) wiedergibt, worin $R^4$ und $R^5$ gemeinsam mit dem Kohlenstoffatom, an das sie gebunden sind, eine Gruppe C = O bilden, $R^2$ auch eine Aldehydgruppe bedeuten kann;

oder $R^2$ eine Hydroxymethylgruppe wiedergibt und -A-

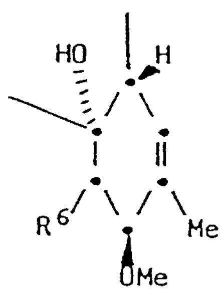

(worin $R^6$ ein Wasserstoffatom oder eine Hydroxygruppe bedeutet) darstellt; mit den Maßgaben, daß, wenn $R^2$ für eine Methylgruppe steht und $R^4$ für eine Methoxygruppe steht, $R^3$ nicht eine Methyl- oder Isopropylgruppe bedeuten kann, und wenn $R^2$ für eine Hydroxymethylgruppe steht und $R^6$ für eine Hydroxygruppe steht, $R^3$ nicht eine Methylgruppe bedeuten kann,
welches Verfahren die Stufe der Kultivierung eines Organismus der Gattung Streptomyces der zur Bildung zumindest einer dieser Verbindungen befähigt ist, wodurch zumindest eine dieser Verbindungen gebildet wird, und gewünschtenfalls die Isolierung dieser Verbindung hieraus umfaßt.

**2.** Verfahren gemäß Anspruch 1, worin $R^3$ in dem Produkt eine Isopropylgruppe ist.

**3.** Verfahren gemäß Anspruch 1, worin in dem Produkt $R^1$ und $R^2$ Methylgruppen bedeuten, $R^3$ eine Isopropylgruppe ist und -A- eine Gruppe (ii) bedeutet, worin $R^4$ für eine Hydroxygruppe steht und $R^5$ ein Wasserstoffatom bedeutet oder $R^4$ und $R^5$ gemeinsam mit dem Kohlenstoffatom, an das sie gebunden sind, für C = O stehen.

**4.** Verfahren gemäß einem der vorhergehenden Ansprüche, worin der Organismus der Gattung Streptomyces die Species Streptomyces thermoarchaensis oder Streptomyces cyaneogriseus noncyanogenus ist.

**5.** Verfahren gemäß Anspruch 4, worin der Organismus NCIB 12015, NCIB 12111, NCIB 12112, NCIB 12113, NCIB 12114, NCIB 12212 oder NCIB 12334 oder Streptomyces cyaneogriseus noncyanogenus NRRL 15773 oder eine Mutante irgendeines dieser Stämme ist.

**6.** Zusammensetzung für die Schädlingskontrolle (zu einer anderen Verwendung als als pharmazeutische Zusammensetzung), enthaltend eine wirksame Menge zumindest einer Verbindung der Formel (I), wie in Anspruch 1 definiert, zusammen mit einem oder mehreren Trägern und/oder Exzipienten.

7. Verfahren zur Bekämpfung von Schädlingen in der Landwirtschaft, im Gartenbau oder der Forstwirtschaft oder in Warenhäusern, Gebäuden oder anderen öffentlichen Plätzen oder Aufenthaltsorten der Schädlinge, das das Aufbringen einer wirksamen Menge einer oder mehrerer Verbindungen der Formel (I), wie in Anspruch 1 definiert, auf Pflanzen oder andere Vegetation oder auf die Schädlinge selbst oder deren Aufenthaltsort umfaßt.

8. Verfahren gemäß Anspruch 7, worin die Schädlinge Insekten, Milben oder Nematodenschädlinge sind.

9. Streptomyces thermoarchaensis NCIB 12212 und dessen Mutanten und dessen genetisches Material, das bei der Synthese der Verbindungen gemäß Anspruch 1 teilhat.

10. Verwendung einer Verbindungen der Formel (I), wie in Anspruch 1 definiert, bei der Herstellung einer Verbindung der nachstehenden Formel (II) (oder eines 5-Ketoderivats hiervon)

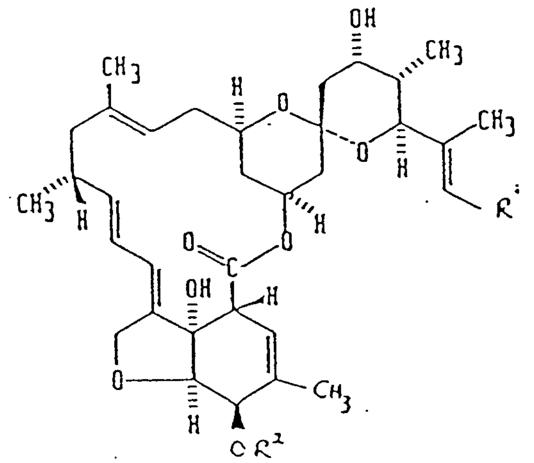

(II)

(worin $R^1$ für eine Methyl-, Ethyl- oder Isopropylgruppe steht und $R^2$ ein Wasserstoffatom oder eine Methylgruppe bedeutet), das die Kultivierung eines geeigneten Streptomyces-Organismus in Anwesenheit der Verbindung der Formel (I), wodurch eine Verbindung der Formel (II) gebildet wird, und gewünschtenfalls die Isolierung dieser Verbindung umfaßt.

11. Verwendung gemäß Anspruch 10 einer Verbindung, wie in Anspruch 3 definiert, bei der Herstellung einer Verbindung der Formel (II), worin $R^1$ eine Isopropylgruppe bedeutet und $R^2$ für ein Wasserstoffatom steht.

12. Verwendung gemäß Anspruch 10 oder 11, worin der Organismus ein Stamm von Streptomyces thermoarchaensis, Streptomyces cyaneogriseus noncyanogenus, Streptomyces avermitilis oder Streptomyces hygroscopicus subsp.aureolacrimosus ist.

13. Verwendung gemäß Anspruch 12, worin der Organismus Streptomyces thermoarchaensis NCIB 12213 oder eine Mutante hiervon, Streptomyces avermitilis ATCC 31272 oder Streptomyces hygroscopicus subsp. aureolacrimosus FERM P1438 ist.

14. Streptomyces thermoarchaensis NCIB 12213 und dessen Mutanten und dessen genetisches Material, das bei der Synthese der Verbindungen der Formel (II), wie in Anspruch 10 definiert, teilhat.

**Revendications**

**Revendications pour les Etats contractants : BE, CH, DE, FR, GB, IT, LI, NL, SE**

1. Composés qui répondent à la formule (I)

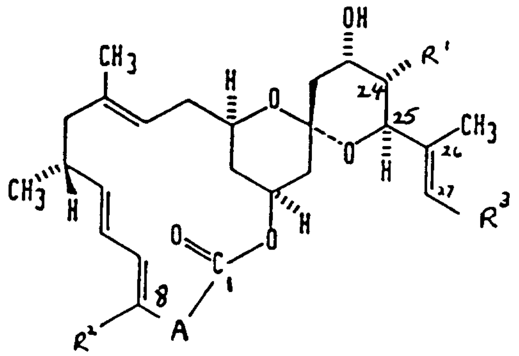

(I)

dans laquelle

$R^1$ représente le radical méthyle,
$R^2$ représente le radical méthyle,
$R^3$ représente le radical méthyle, éthyle ou isopropyle et
-A- représente un noyau tel que représenté ci-dessous

(i) 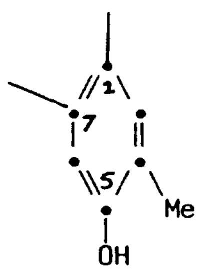 ou (ii) 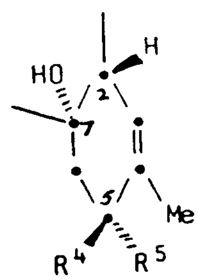

(où $R^4$ représente un radical hydroxyle ou méthoxy et $R^5$ représente un atome d'hydrogène, ou bien $R^4$ et $R^5$ forment, ensemble avec l'atome de carbone auquel ils sont attachés, un groupe C = O); ou bien, lorsque -A-représente le noyau (ii) dans lequel $R^4$ et $R^5$ forment, ensemble, avec l'atome de carbone auquel ils sont attachés, un groupe C = O, alors $R^2$ peut également représenter un radical aldéhydo;
ou bien $R^2$ représente un radical hydroxyméthyle et -A-représente un noyau

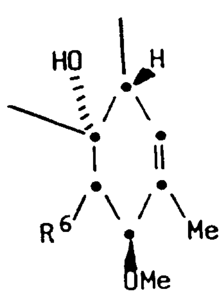

(où $R^6$ représente un atome d'hydrogène ou le radical hydroxyle);
avec les conditions que lorsque $R^2$ représente le radical méthyle et $R^4$ représente le radical méthoxy, alors $R^3$ ne peut pas représenter un radical méthyle ou isopropyle et lorsque $R^2$ représente le radical

hydroxyméthyle et $R^6$ représente le radical hydroxyle, alors $R^3$ ne peut pas représenter de radical méthyle.

**2.** Composés suivant la revendication 1 de la formule (I) dans laquelle $R^3$ représente le radical isopropyle.

**3.** Composés suivant la revendication 1 de la formule (I) dans laquelle $R^1$ et $R^2$ représentent des radicaux méthyle, $R^3$ représente le radical isopropyle et -A-représente un groupe (ii) dans lequel $R^3$ représente le radical hydroxyle et $R^5$ représente un atome d'hydrogène, ou bien $R^4$ et $R^5$ forment, ensemble avec l'atome de carbone auquel ils sont attachés, un groupe C = O.

**4.** Composition destinée à l'usage en médecine humaine, qui contient une proportion efficace d'au moins un composé suivant la revendication 1, ainsi qu'un ou plusieurs excipients et/ou véhicules.

**5.** Composition destinée à l'usage en médecine vétérinaire, qui contient une proportion efficace d'au moins un composé suivant la revendication 1, ainsi qu'un ou plusieurs excipients et/ou véhicules.

**6.** Composition pour lutter contre les organismes nuisibles ou pesticide, qui contient une quantité efficace d'au moins un composé suivant la revendication 1, ainsi qu'un ou plusieurs excipients et/ou véhicules.

**7.** Procédé pour combattre des organismes nuisibles dans le domaine agricole, horticole ou forestier, ou dans des entrepôts, magasins, édifices, ou d'autres places publiques, ou d'autres endroits où logent les organismes nuisibles, caractérisé en ce que l'on applique aux plantes ou à toute autre végétation, ou aux organismes nuisibles eux-mêmes, ou en un endroit où ils logent, une quantité efficace d'un ou plusieurs composés suivant la revendication 1.

**8.** Procédé suivant la revendication 7, caractérisé en ce que les organismes nuisibles sont des insectes, des acariens ou des nématodes.

**9.** Procédé de préparation d'un composé suivant la revendication 1, caractérisé en ce qu'il comprend l'étape consistant à cultiver un organisme du genre Streptomyces capable de produire au moins l'un desdits composés, si bien qu'au moins l'un desdits composés est produit et, si on le souhaite, on isole ledit ou lesdits composés.

**10.** Procédé suivant la revendication 9, caractérisé en ce que l'organisme du genre Streptomyces est l'espèce Streptomyces thermoarchaensis ou Streptomyces cyaneogriseus noncyanogenus.

**11.** Procédé suivant la revendication 9, caractérisé en ce que ledit organisme est le suivant Streptomyces thermoarchaensis NCIB 12015, NCIB 12111, NCIB 12112, NCIB 12113, NCIB 12114, NCIB 12212 ou NCIB 12334 ou Streptomyces cyaneogriseus noncyanogenus NRRL 15773, ou un mutant de n'importe laquelle de ces souches.

**12.** Streptomyces thermoarchaensis NCIB 12212 et ses mutants et la matière génétique de ceux-ci qui participe à la synthèse de composés suivant la revendication 1.

**13.** Utilisation d'un composé de la formule (I) selon la revendication 1 en vue de la préparation d'un composé de la formule (II) ci-dessous (ou d'un dérivé du type 5-céto d'un tel composé)

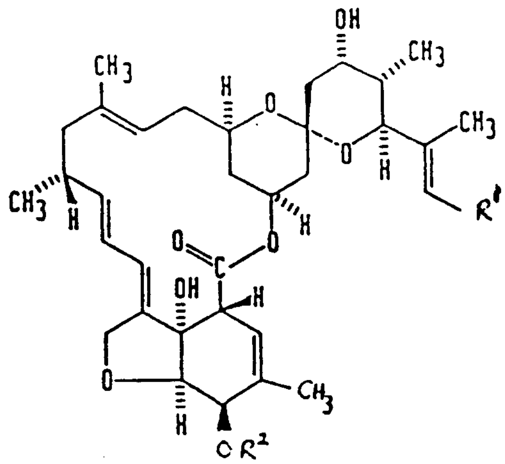

(II)

(dans laquelle $R^1$ représente le radical méthyle, éthyle ou isopropyle et $R^2$ représente un atome d'hydrogène ou le radical méthyle), caractérisé en ce que l'on cultive un organisme du genre Streptomyces approprié en présence d'un composé de la formule (I), de manière à produire un composé de la formule (IL) et, si on le souhaite, on isole le composé en question.

**14.** Utilisation suivant la revendication 13 d'un composé suivant la revendication 3 pour la production d'un composé de la formule (II) dans laquelle $R^1$ représente le radical isopropyle et $R^2$ représente un atome d'hydrogène.

**15.** Utilisation suivant la revendication 13 ou la revendication 14, caractérisé en ce que l'organisme est une souche de Streptomyces thermoarchaensis, Streptomyces cyaneogriseus noncyanogenus, Streptomyces avermitilis ou Streptomyces hygroscopicus subsp. aureolacrimosus.

**16.** Utilisation suivant la revendication 15, caractérisé en ce que l'organisme est Streptomyces thermoarchaensis NCIB 12213, ou un mutant de celui-ci, Streptomyces avermitilis ATCC 31272 ou Streptomyces hygroscopicus subsp. aureolacrimosus FERM P 1438.

**17.** Streptomyces thermoarchaensis NCIB 12213 et ses mutants et la matière génétique de ceux-ci qui participe à la synthèse de composés de la formule (II) telle que définie dans la revendication 13.

**Revendications pour les Etats contractants suivants : AT, ES**

**1.** Procédé de préparation de composés répondant à la formule (I)

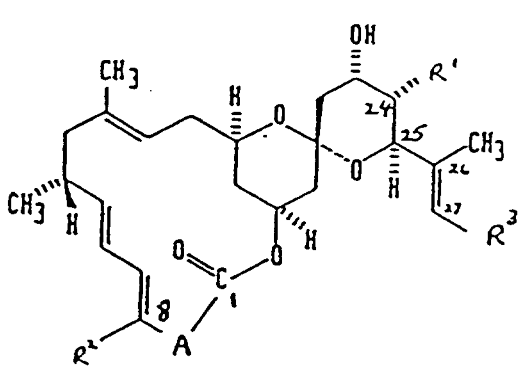

(I)

dans laquelle

$R^1$ représente le radical méthyle,

$R^2$ représente le radical méthyle,

$R^3$ représente le radical méthyle, éthyle ou isopropyle et
-A- représente un noyau tel que représenté ci-dessous

(i) 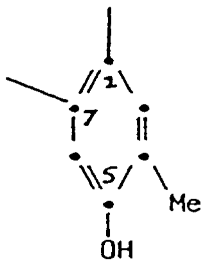 ou (ii) 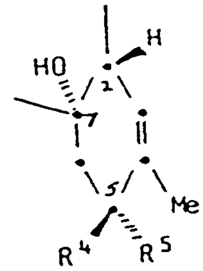

(où $R^4$ représente un radical hydroxyle ou méthoxy et $R^5$ représente un atome d'hydrogène, ou bien $R^4$ et $R^5$ forment, ensemble avec l'atome de carbone auquel ils sont attachés, un groupe C = O); ou bien, lorsque -A-représente le noyau (ii) dans lequel $R^4$ et $R^5$ forment, ensemble avec l'atome de carbone auquel ils sont attachés, un groupe C = O, alors $R^2$ peut également représenter un radical aldéhydo;
ou bien $R^2$ représente un radical hydroxyméthyle et -A-représente un noyau

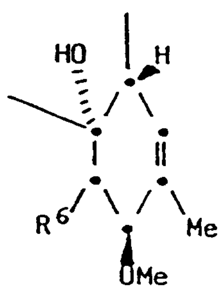

(où $R^6$ représente un atome d'hydrogène ou le radical hydroxyle);
avec les conditions que lorsque $R^2$ représente le radical méthyle et $R^4$ représente le radical méthoxy, alors $R^3$ ne peut pas représenter un radical méthyle ou isopropyle et lorsque $R^2$ représente le radical hydroxyméthyle et $R^6$ représente le radical hydroxyle, alors $R^3$ ne peut pas représenter de radical méthyle, caractérisé en ce qu'il comprend l'étape consistant à cultiver un organisme du genre Streptomyces capable de produire au moins l'un desdits composés, si bien qu'au moins l'un desdits composés est produitet, si on le souhaite, on isole ledit ou lesdits composés.

2. Procédé suivant la revendication 1, caractérisé en ce que $R^3$ représente le radical isopropyle dans le produit.

3. Procédé suivant la revendication 1, caractérisé en ce que, dans le produit, $R^1$ et $R^2$ représentent des radicaux méthyle, $R^3$ représente le radical isopropyle et -A- représente un groupe (ii) dans lequel $R^3$ représente le radical hydroxyle et $R^5$ représente un atome d'hydrogène, ou bien $R^4$ et $R^5$ forment, ensemble avec l'atome de carbone auquel ils sont attachés, un groupe C = O.

4. Procédé suivant l'une quelconque des revendications précédentes, caractérisé en ce que l'organisme du genre Streptomyces est l'espèce Streptomyces thermoarchaensis ou Streptomyces cyaneogriseus non cyanogenus.

5. Procédé suivant la revendication 4, caractérisé en ce que ledit organisme est le suivant Streptomyces thermoarchaensis NCIB 12015, NCIB 12111, NCIB 12112, NCIB 12113, NCIB 12114, NCIB 12212 ou NCIB 12334 ou Streptomyces cyaneogriseus noncyanogenus NRRL 15773, ou un mutant de n'importe laquelle de ces souches.

6. Composition pesticide ou de luttre contre les organismes nuisibles (à utiliser autrement que sous forme d'une composition pharmaceutique), qui contient une proportion efficace d'au moins un composé de la formule (I) telle que définie dans la revendication 1, ainsi qu'un ou plusieurs excipients et/ou véhicules.

7. Procédé pour combattre des organismes nuisibles dans le domaine agricole, horticole ou forestier, ou dans des entrepôts, magasins, édifices, ou d'autres places publiques, ou d'autres endroits où logent les organismes nuisibles, caractérisé en ce que l'on applique aux plantes ou à toute autre végétation, ou aux organismes nuisibles eux-mêmes, ou en un endroit où ils logent, une quantité efficace d'un ou plusieurs composés suivant la revendication 1.

8. Procédé suivant la revendication 7, caractérisé en ce que les organismes nuisibles sont des insectes, des acariens ou des nématodes.

9. Streptomyces thermoarchaensis NCIB 12212 et ses mutants et la matière génétique de ceux-ci qui participe à la synthèse de composés suivant la revendication 1.

10. Utilisation d'un composé de la formule (I) selon la revendication 1 en vue de la préparation d'un composé de la formule (II) ci-dessous (ou d'un dérivé du type 5-céto d'un tel composé)

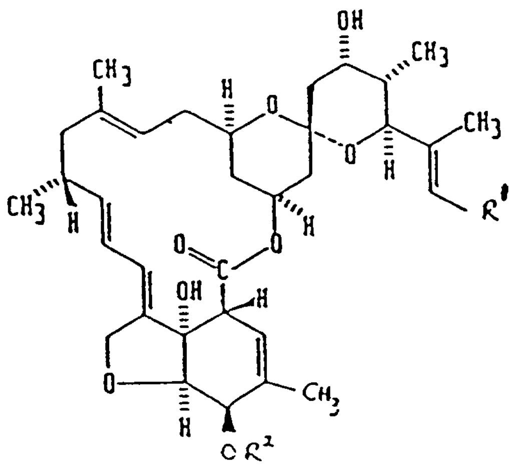

(II)

(dans laquelle $R^1$ représente le radical méthyle, éthyle ou isopropyle et $R^2$ représente un atome d'hydrogène ou le radical méthyle), caractérisé en ce que l'on cultive un organisme du genre Streptomyces approprié en présence d'un composé de la formule (I), de manière à produire un composé de la formule (II) et, si on le souhaite, on isole le composé en question.

11. Utilisation suivant la revendication 10 d'un composé tel que défini dans la revendication 3 pour la production d'un composé d ela formule (II) dans laquelle $R^1$ représente le radical isopropyle et $R^2$ représente un atome d'hydrogène.

12. Utilisation suivant la revendication 10 ou la revendication 11, caractérisé en ce que l'organisme est une souche de Streptomyces thermoarchanesis, Streptomyces cyaneogriseus non cyanogenus, Streptomyces avermitilis ou Streptomyces hygroscopicus subsq. aureolacrimosus.

13. Utilisation suivant la revendication 12, caractérisé en ce que l'organisme est Streptomyces thermoarchaensis NCIB 12213, ou un mutant de celui-ci, Streptomyces avermitilis ATCC 31272 ou Streptomyces hygroscopicus subsq. aureolacrimus FERM P 1438.

14. Streptomyces thermoarchaensis NCIB 12213 et ses mutants et la matière génétique de ceux-ci qui participe à la synthèse de composés de la formule (II) telle que définie dans la revendication 10.